# EUROPEAN PATENT SPECIFICATION

(11) **EP 2 804 598 B1**
(45) Date of publication and mention of the grant of the patent: **15.05.2019**
(21) Application number: 13738597.7
(22) Date of filing: 17.01.2013
(51) Int. Cl.: A61K 31/192, A61P 21/00, C12Q 1/68, G01N 33/15, G01N 33/50

(54) **PROPHYLACTIC AND THERAPEUTIC DRUG FOR AMYOTROPHIC LATERAL SCLEROSIS AND METHOD OF SCREENING THEREFOR**
PROPHYLAKTISCHES UND THERAPEUTISCHES MITTEL FÜR AMYOTROPHE LATERALSKLEROSE UND VERFAHREN ZUM SCREENING DANACH
MÉDICAMENT PROPHYLACTIQUE ET THÉRAPEUTIQUE POUR LA SCLÉROSE LATÉRALE AMYOTROPHIQUE, ET PROCÉDÉ DE CRIBLAGE ASSOCIÉ

(30) Priority: 17.01.2012 US 201261587323 P
(43) Date of publication of application: 26.11.2014
(73) Proprietor: Kyoto University, Kyoto-shi, Kyoto 606-8501 (JP)
(72) Inventor: INOUE, Haruhisa, Kyoto-shi Kyoto 606-8501 (JP); EGAWA, Naohiro, Kyoto-shi Kyoto 606-8501 (JP); KITAOKA, Shiho, Kyoto-shi Kyoto 606-8501 (JP); TSUKITA, Kayoko, Kyoto-shi Kyoto 606-8501 (JP)
(74) Representative: J A Kemp
(86) International application number: PCT/JP2013/051358
(87) International publication number: WO 2013/108926

(56) References cited:
- JP-A- 2005 306 794
- E ESPOSITO ET AL: "A review of specific dietary antioxidants and the effects on biochemical mechanisms related to neurodegenerative processes", NEUROBIOLOGY OF AGING., vol. 23, no. 5, 1 September 2002 (2002-09-01), pages 719-735, XP055232352, US ISSN: 0197-4580, DOI: 10.1016/S0197-4580(02)00078-7
- KUBO I ET AL: "Antioxidant activity of anacardic acids", FOOD CHEMISTRY, ELSEVIER LTD, NL, vol. 99, no. 3, 1 January 2006 (2006-01-01), pages 555-562, XP027989453, ISSN: 0308-8146 [retrieved on 2006-01-01]
- KUBO I ET AL.: 'Antioxidant activity of anacardic acids' FOOD CHEMISTRY vol. 99, no. 3, 2006, pages 555 - 562, XP005434506
- DUAN W ET AL.: 'MG132 enhances neurite outgrowth in neurons overexpressing mutant TAR DNA-binding protein-43 via increase of HO-1.' BRAIN RES. vol. 1397, 10 May 2011, pages 1 - 9, XP055081491
- WHITE A ET AL.: 'TDP-43 aggregation during chronic oxidative stress is mediated by c-jun n-terminal kinase and can be inhibited bya potentially' ALZHEIMER'S AND DEMENTIA vol. 7, no. 4, July 2011, page S118, XP055159140
- COLOMBRITA C ET AL.: 'TDP-43 is recruited to stress granules in conditions of oxidative insult.' J NEUROCHEM. vol. 111, no. 4, 16 September 2009, pages 1051 - 1061, XP055081492

### SEQUENCE LISTING

<110> Kyoto University
<120> Prophylactic and Therapeutic Drug for Amyotrophic Lateral Sclerosis and Method of Screening Therefor
<130> 091979
<150> US 61/587323
   <151> 2012-01-17
<160> 2
<170> Patent In version 3.5
<210> 1
   <211> 1206
   <212> DNA
   <213> Artificial Sequence
<220>
   <223> TDP-43 fragment
<220>
   <221> CDS
   <222> (1).. (1206)
<400> 1
   atg tct gaa tat att cgg gta acc gaa gat gag aac gat gag ccc att      48
   Met Ser Glu Tyr Ile Arg Val Thr Glu Asp Glu Asn Asp Glu Pro Ile
   1               5                   10                  15
   gaa ata cca tcg gaa gac gat ggg acg gtg ctg ctc tcc acg gtt aca      96
   Glu Ile Pro Ser Glu Asp Asp Gly Thr Val Leu Leu Ser Thr Val Thr
               20                  25                  30
   ggg ctt cgc tac agg aat cca gtg tct cag tgt atg aga ggt gtc cgg      144
   Gly Leu Arg Tyr Arg Asn Pro Val Ser Gln Cys Met Arg Gly Val Arg
           35                  40                  45
   ctg gta gaa gga att ctg cat gcc cca gat gct ggc tgg gga aat ctg      192
   Leu Val Glu Gly Ile Leu His Ala Pro Asp Ala Gly Trp Gly Asn Leu
       50                  55                  60
   gtg tat gtt gtc aac tat cca aaa gat aac aaa aga aaa atg gat gag      240
   Val Tyr Val Val Asn Tyr Pro Lys Asp Asn Lys Arg Lys Met Asp Glu
   65                  70                  75                  80
   aca gat gct tca tca gca gtg aaa gtg aaa aga gca gtc cag aaa aca      288
   Thr Asp Ala Ser Ser Ala Val Lys Val Lys Arg Ala Val Gln Lys Thr
                   85                  90                  95
   tcc gat tta ata gtg ttg ggt ctc cca tgg aaa aca acc gaa cag gac      336
   Ser Asp Leu Ile Val Leu Gly Leu Pro Trp Lys Thr Thr Glu Gln Asp
               100                 105                 110
   ctg aaa gag tat ttt agt acc ttt gga gaa gtt ctt atg gtg cag gtc      384
   Leu Lys Glu Tyr Phe Ser Thr Phe Gly Glu Val Leu Met Val Gln Val
           115                 120                 125
   aag aaa gat ctt aag act ggc ttt gtt cgt ttt acg gaa tat gaa aca      432
   Lys Lys Asp Leu Lys Thr Gly Phe Val Arg Phe Thr Glu Tyr Glu Thr
       130                 135                 140
   caa gtg aaa gta atg tca cag cga cat atg ata gat gga cga tgg tgt      480
   Gln Val Lys Val Met Ser Gln Arg His Met Ile Asp Gly Arg Trp Cys
   145                 150                 155                 160
   gac tgc aaa ctt cct aat tct aag caa agc caa gat gag cct ttg aga      528
   Asp Cys Lys Leu Pro Asn Ser Lys Gln Ser Gln Asp Glu Pro Leu Arg
                   165                 170                 175
   agc aga aaa gtg ttt gtg ggg cgc tgt aca gag gac atg act gag gat      576
   Ser Arg Lys Val Phe Val Gly Arg Cys Thr Glu Asp Met Thr Glu Asp
               180                 185                 190
   gag ctg cgg gag ttc ttc tct cag tac ggg gat gtg atg gat gtc ttc      624
   Glu Leu Arg Glu Phe Phe Ser Gln Tyr Gly Asp Val Met Asp Val Phe
           195                 200                 205
   atc ccc aag cca ttc agg gcc ttt gcc ttt gtt aca ttt gca gat gat      672
   Ile Pro Lys Pro Phe Arg Ala Phe Ala Phe Val Thr Phe Ala Asp Asp
       210                 215                 220
   cag att gcg cag tct ctt tgt gga gag gac ttg atc att aaa gga atc      720
   Gln Ile Ala Gln Ser Leu Cys Gly Glu Asp Leu Ile Ile Lys Gly Ile
   225                 230                 235                 240
   agc gtt cat ata tcc aat gcc gaa cct aag cac aat agc aat aga cag      768
   Ser Val His Ile Ser Asn Ala Glu Pro Lys His Asn Ser Asn Arg Gln
                   245                 250                 255
   tta gaa aga agt gga aga ttt ggt ggt aat cca ggt ggc ttt ggg aat      816
   Leu Glu Arg Ser Gly Arg Phe Gly Gly Asn Pro Gly Gly Phe Gly Asn
               260                 265                 270
   cag ggt gga ttt ggt aat agc aga ggg ggt gga gct ggt ttg gga aac      864
   Gln Gly Gly Phe Gly Asn Ser Arg Gly Gly Gly Ala Gly Leu Gly Asn
           275                 280                 285
   aat caa ggt agt aat atg ggt ggt ggg atg aac ttt ggt gcg ttc agc      912
   Asn Gln Gly Ser Asn Met Gly Gly Gly Met Asn Phe Gly Ala Phe Ser
       290                 295                 300
   att aat cca gcc atg atg gct gcc gcc cag gca gca cta cag agc agt      960
   Ile Asn Pro Ala Met Met Ala Ala Ala Gln Ala Ala Leu Gln Ser Ser
   305                 310                 315                 320
   tgg ggt atg atg ggc atg tta gcc agc cag cag aac cag tca ggc cca     1008
   Trp Gly Met Met Gly Met Leu Ala Ser Gln Gln Asn Gln Ser Gly Pro
                   325                 330                 335
   tcg ggt aat aac caa aac caa ggc aac atg cag agg gag cca aac cag     1056
   Ser Gly Asn Asn Gln Asn Gln Gly Asn Met Gln Arg Glu Pro Asn Gln
               340                 345                 350
   gcc ttc ggt tct gga aat aac tct tat agt ggc tct aat tct ggt gca     1104
   Ala Phe Gly Ser Gly Asn Asn Ser Tyr Ser Gly Ser Asn Ser Gly Ala
           355                 360                 365
   gca att ggt tgg gga tca gca tcc aat gca ggg tcg ggc agt ggt ttt     1152
   Ala Ile Gly Trp Gly Ser Ala Ser Asn Ala Gly Ser Gly Ser Gly Phe
       370                 375                 380
   aat gga ggc ttt ggc tca agc atg gat tct aag tct tct ggc tgg gga        1200
   Asn Gly Gly Phe Gly Ser Ser Met Asp Ser Lys Ser Ser Gly Trp Gly
   385                 390                 395                 400
   atg tag                                                                1206
   Met
<210> 2
   <211> 401
   <212> PRT
   <213> Artificial Sequence
<220>
   <223> Synthetic Construct
<400> 2
   Met Ser Glu Tyr Ile Arg Val Thr Glu Asp Glu Asn Asp Glu Pro Ile
   1               5                   10                  15
   Glu Ile Pro Ser Glu Asp Asp Gly Thr Val Leu Leu Ser Thr Val Thr
               20                  25                  30
   Gly Leu Arg Tyr Arg Asn Pro Val Ser Gln Cys Met Arg Gly Val Arg
           35                  40                  45
   Leu Val Glu Gly Ile Leu His Ala Pro Asp Ala Gly Trp Gly Asn Leu
       50                  55                  60
   Val Tyr Val Val Asn Tyr Pro Lys Asp Asn Lys Arg Lys Met Asp Glu
   65                  70                  75                  80
   Thr Asp Ala Ser Ser Ala Val Lys Val Lys Arg Ala Val Gln Lys Thr
                   85                  90                  95
   Ser Asp Leu Ile Val Leu Gly Leu Pro Trp Lys Thr Thr Glu Gln Asp
               100                 105                 110
   Leu Lys Glu Tyr Phe Ser Thr Phe Gly Glu Val Leu Met Val Gln Val
           115                 120                 125
   Lys Lys Asp Leu Lys Thr Gly Phe Val Arg Phe Thr Glu Tyr Glu Thr
       130                 135                 140
   Gln Val Lys Val Met Ser Gln Arg His Met Ile Asp Gly Arg Trp Cys
   145                 150                 155                 160
   Asp Cys Lys Leu Pro Asn Ser Lys Gln Ser Gln Asp Glu Pro Leu Arg
                   165                 170                 175
   Ser Arg Lys Val Phe Val Gly Arg Cys Thr Glu Asp Met Thr Glu Asp
   180 185 190
   Glu Leu Arg Glu Phe Phe Ser Gln Tyr Gly Asp Val Met Asp Val Phe
   195 200 205
   Ile Pro Lys Pro Phe Arg Ala Phe Ala Phe Val Thr Phe Ala Asp Asp
   210 215 220
   Gln Ile Ala Gln Ser Leu Cys Gly Glu Asp Leu Ile Ile Lys Gly Ile
   225 230 235 240
   Ser Val His Ile Ser Asn Ala Glu Pro Lys His Asn Ser Asn Arg Gln
   245 250 255
   Leu Glu Arg Ser Gly Arg Phe Gly Gly Asn Pro Gly Gly Phe Gly Asn
   260 265 270
   Gln Gly Gly Phe Gly Asn Ser Arg Gly Gly Gly Ala Gly Leu Gly Asn
   275 280 285
   Asn Gln Gly Ser Asn Met Gly Gly Gly Met Asn Phe Gly Ala Phe Ser
   290 295 300
   Ile Asn Pro Ala Met Met Ala Ala Ala Gln Ala Ala Leu Gln Ser Ser
   305 310 315 320
   Trp Gly Met Met Gly Met Leu Ala Ser Gln Gln Asn Gln Ser Gly Pro
   325 330 335
   Ser Gly Asn Asn Gln Asn Gln Gly Asn Met Gln Arg Glu Pro Asn Gln
   340 345 350
   Ala Phe Gly Ser Gly Asn Asn Ser Tyr Ser Gly Ser Asn Ser Gly Ala
   355 360 365
   Ala Ile Gly Trp Gly Ser Ala Ser Asn Ala Gly Ser Gly Ser Gly Phe
   370 375 380
   Asn Gly Gly Phe Gly Ser Ser Met Asp Ser Lys Ser Ser Gly Trp Gly
   385 390 395 400
   Met

## Description

### Background of the Invention

Amyotrophic lateral sclerosis (hereinafter sometimes to be referred to as ALS) is a motor neuron disease of poor prognosis, which develops at middle ages and thereafter and causes progressive paralysis of skeletal muscles. It is designated as a disease in the specific disease treatment research program sponsored by the Ministry of Health, Labor and Welfare of Japan. More than about 90% of the cases of ALS are sporadic, and a 43-kDa TAR DNA-binding protein (TDP-43) has recently been identified as a component of ubiquitin-positive inclusions seen in the lower motor neurons of the sporadic ALS patients, and drawing attention as a pathogenic gene (1). On the other hand, the remaining 10% are familial cases, in which point mutation of genes such as Cu/Zn superoxide dismutase (SOD1) gene, TDP-43 gene and the like has been reported. In this case, to explain the causal factor in the latter cases, the gain-of-toxic function theory is likely wherein motor neuron death is caused by the cytotoxicity newly gained by mutated SOD1 (2).

The only currently commercially available therapeutic drug for ALS is riluzole (Rilutek™, Aventis), a glutamate receptor antagonist possessing glutamate suppressing action (3).

On the other hand, pathogenic cells obtained by establishing induced pluripotent stem cells (iPS cells) from the cells derived from patient and inducing differentiation thereof thereinto, by using a reprogramming technique for establishing pluripotent cells from human dermal fibroblasts, are considered to enable reproduction of the pathologic condition in vitro. In fact, the above-described method was successfully applied to generate iPS cells from ALS patient and differentiate into neurons (4).

However, there is no report on an index useful for searching a therapeutic drug for ALS, using a neuron derived from an iPS cell, and therefore, no therapeutic drugs for ALS have been discovered to date.

### Cited references:

1. Neumann M, et al., Science. 2006, 314: 130-3.
2. Bruijn, L.I., et al., Annu. Rev. Neurosci. 2004, 27: 723-749
3. AU 666150 B2
4. Dimos JT, et al., Science. 2008, 321: 1218-21

### SUMMARY OF THE DISCLOSURE

Described herein is a prophylactic and/or therapeutic agent for ALS, and a method of screening for a drug useful for the prophylaxis and/or treatment of ALS (hereinafter sometimes to be referred to as a prophylactic and/or therapeutic drug for ALS). It is therefore a problem to find a novel index useful for screening for a prophylactic and/or therapeutic drug for ALS and a method of reproducing, *in vitro,* a phenomenon specific to ALS, as well as a prophylactic and/or therapeutic drug for ALS, which utilizes a screening system using said index and reproducing method.

To solve this problem, the present inventors have established iPS cells from fibroblasts of an ALS patient, induced then to differentiate into motor neurons (MNs), compared the motor neurons derived from the ALS patient with motor neurons derived from a healthy person, and found a gene showing a different expression level. Furthermore, they have found that an ALS specific phenomenon can be induced *in vitro* by adding arsenite to the induced motor neurons. Moreover, they have found using such index that an anacardic acid derivative can be a prophylactic or therapeutic drug for ALS, which resulted in the completion of the present invention.

Accordingly, the present invention provides the following.
[1] An anacardic acid derivative for use in the prophylaxis and/or treatment of amyotrophic lateral sclerosis,
   wherein the anacardic acid derivative is a compound represented by the formula 1: 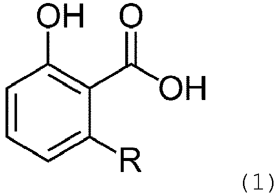 wherein R is C10-17 saturated or unsaturated hydrocarbon group.
[2] The anacardic acid derivative for use according to [1], wherein the amyotrophic lateral sclerosis is sporadic amyotrophic lateral sclerosis.
[3] The anacardic acid derivative for use according to [1], wherein the amyotrophic lateral sclerosis is accompanied by a mutation of TDP-43.

Accordingly the present invention provides an anacardic acid derivative for use in a method to prevent and treat ALS. Also described is a method to screen for a prophylactic or therapeutic drug for ALS using a neuron differentiated from an iPS cell. The present disclosure is therefore highly useful for preventing and treating ALS, and developing a prophylactic or therapeutic drug for ALS.

### BRIEF DESCRIPTION OF THE DRAWINGS

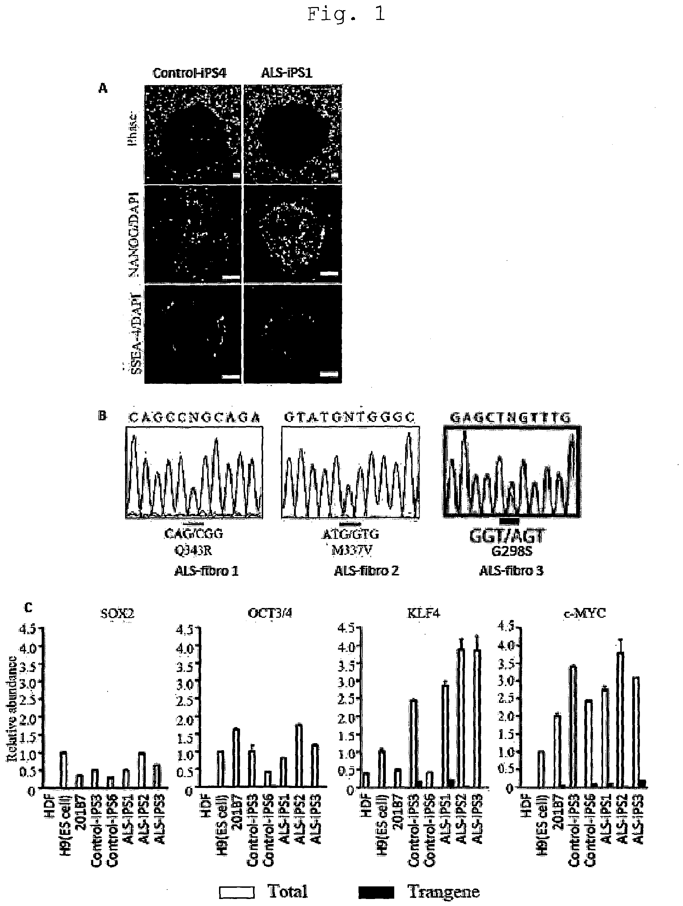

Fig. 1A shows microscopic images of control iPS cell (Control-iPS4) and ALS-derived iPS cell (ALS-iPS1), and immunostaining images using anti-Nanog antibody and anti-SSEA-4 antibody. Fig. 1B shows the results of sequence analysis of the TDP-43 gene locus of the ALS patients-derived fibroblasts. In ALS-fibro 1, the 343rd glutamine is mutated to arginine and, in ALS-fibro 2, the 337th methionine is mutated to valine and, in ALS-fibro 3, the 289th methionine is mutated to serine. Fig. 1C shows the results of the amount of total mRNA and exogeneous mRNA of SOX2, OCT3/4, KLF4 and c-MYC in iPS cell produced using a retrovirus, which were measured by quantitative PCR.

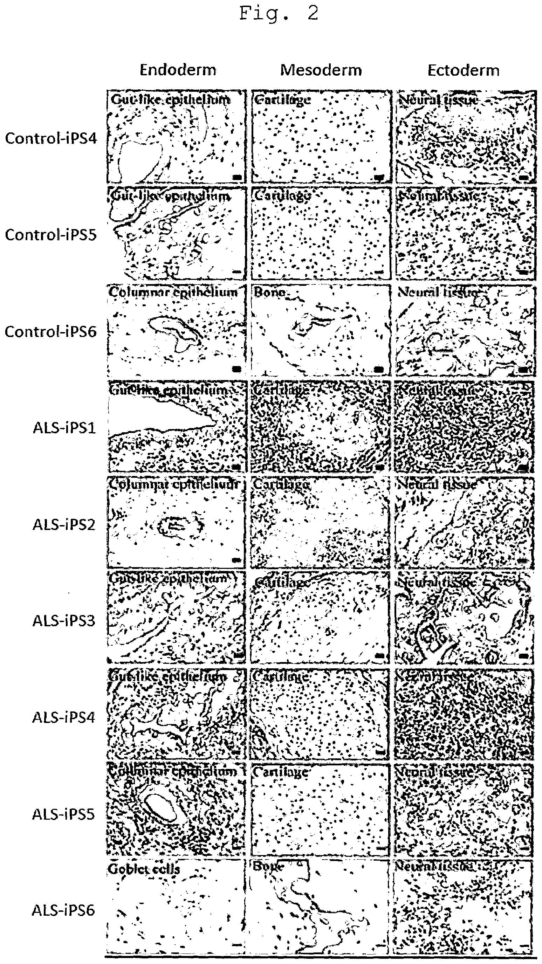

Fig. 2 shows HE stained image of teratoma generated from each established iPS cell, and shows cells having shapes specific to endoderm, mesoderm and ectoderm.

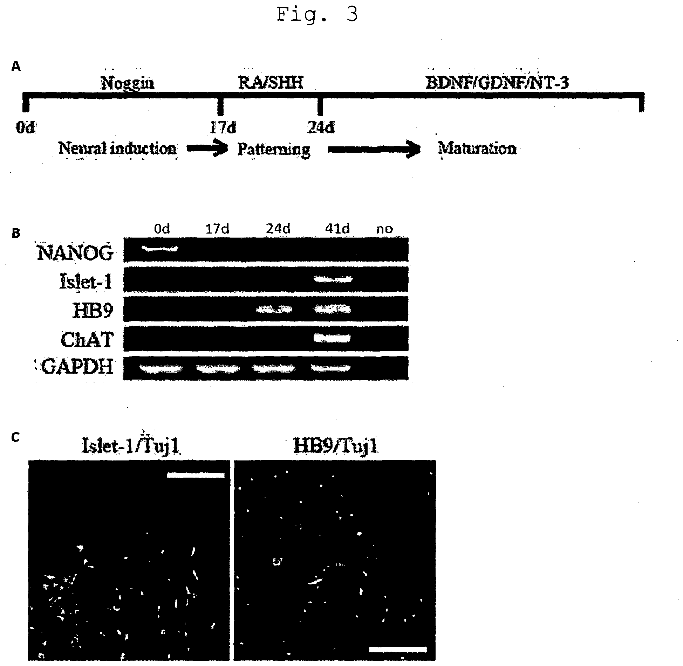

Fig. 3A shows the protocol of neural induction by an adherent culture method. Fig. 3B shows the results of confirmed expression of mRNAs of NANOG, Islet-1, HB9 and ChAT in neurons induced by the adherent culture method. Fig. 3C shows fluorescence images of neurons induced by the adherent culture method and immunostained with anti-Islet-1 antibody, anti-HB9 antibody and anti-Tuj1 antibody.

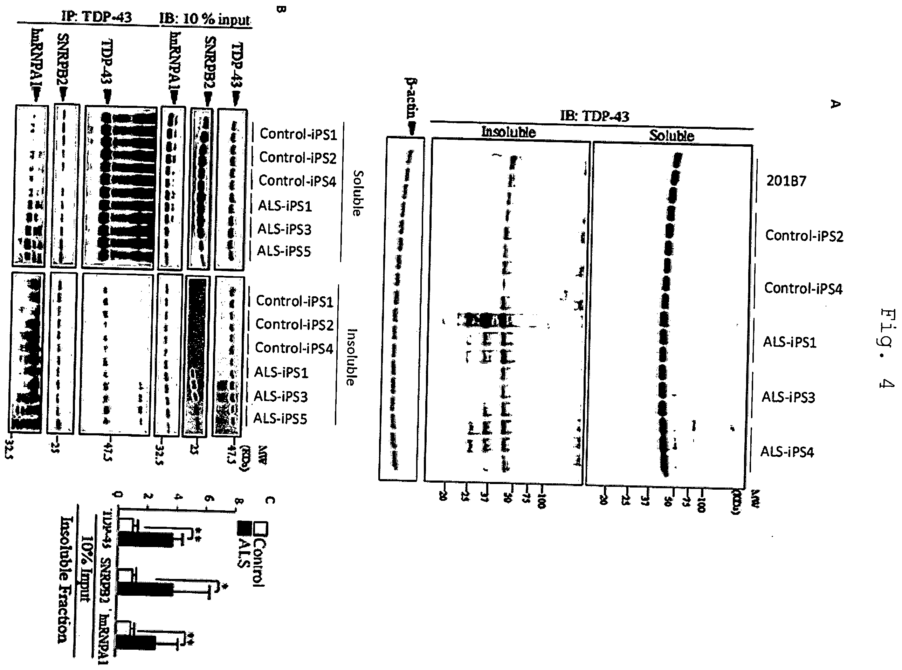

Fig. 4A shows the results of three measurements of TDP-43 protein contained in soluble fractions and insoluble fractions obtained by dissolving neurons derived from 201B7, Control-iPS2, Control-iPS4, ALS-iPS1, ALS-iPS3 and ALS-iPS4 in 1% Triton X-100 solution. Fig. 4B shows the results of the measurement of the protein amount of TDP-43, SNRPB2, hnRNPA1 in soluble fractions and insoluble fractions obtained by dissolving neurons derived from each iPS cell in 1% Triton X-100 solution (IB), and the results of the measurement of the protein amount of TDP-43, SNRPB2, hnRNPA1 in a component precipitated with anti-TDP-43 antibody (IP). Fig. 4C is a graph showing the quantified protein amount of TDP-43, SNRPB2, hnRNPA1 in insoluble fractions.

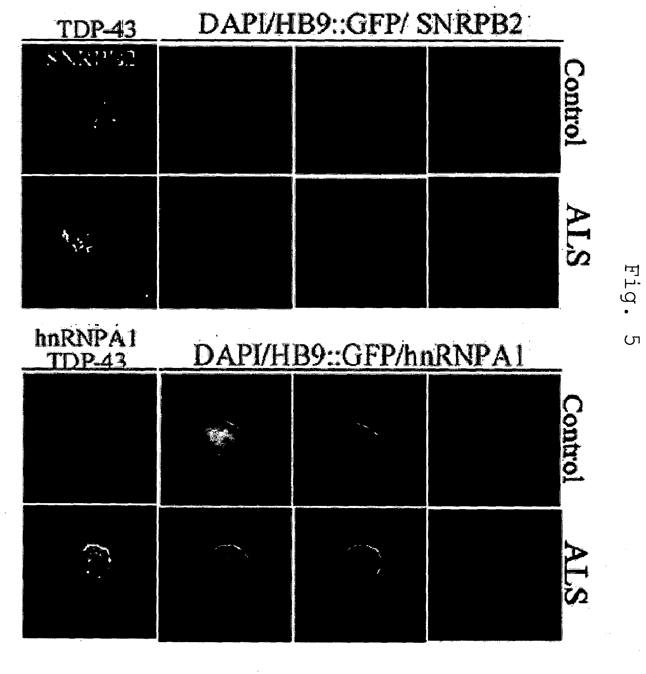

Fig. 5 shows stained images of SNRPB2 (left panel) and hnRNPA1 (right panel) of iPS cell-derived neurons introduced with HB9::GFP. The bottom panel shows co-stained images of TDP-43 and SNRPB2 (left panel), and TDP-43 and hnRNPA1 (right panel).

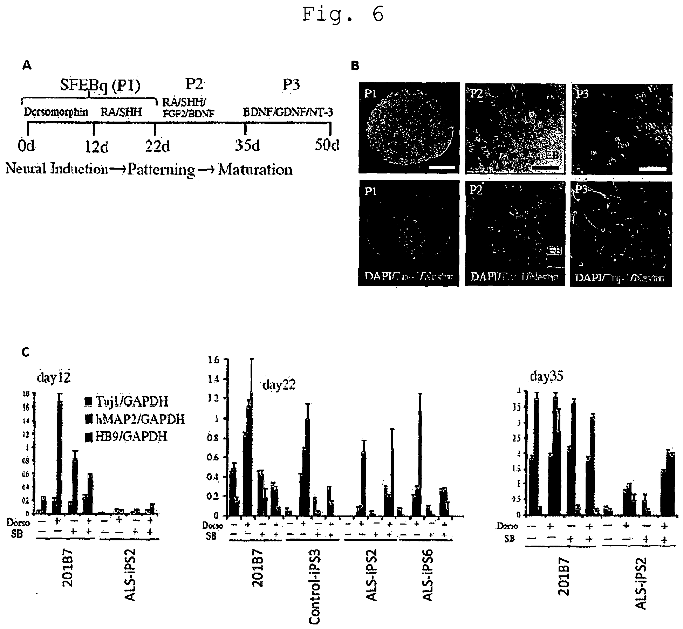

Fig. 6A shows the protocol of neural induction by a modified SFEBq method. Fig. 6B shows microscopic images (upper panel) and co-stained images of Tuj1 and Nestin (bottom panel) 22 days later (P1: left), 35 days later (P2: middle) and 50 days later (P3: right). Fig. 6C shows the results of expression levels of Tuj1, hMAP2 and HB9 in the neurons (day 12, day 22 and day 35) of 201B7, Control-iPS3, ALS-iPS2 and ALS-iPS6 generated in the presence or absence of Dorsomorphin or SB431542, as measured by quantitative PCR.

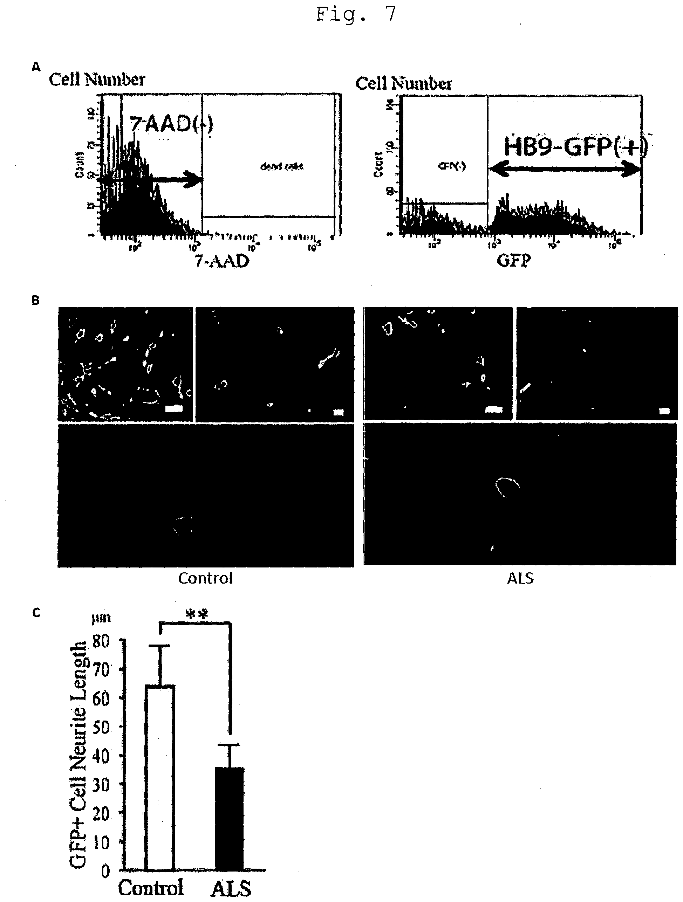

Fig. 7A shows the results of 2 repeats of sorting of iPS cell-derived neurons introduced with HB9::GFP. Fig. 7B shows fluorescence microscopic images of GFP-positive cells of neurons (control and ALS) derived from each iPS cell and introduced with HB9::GFP. Fig. 7C shows the measurement results of the length of neurite of neurons derived from each iPS cell and introduced with HB9::GFP.

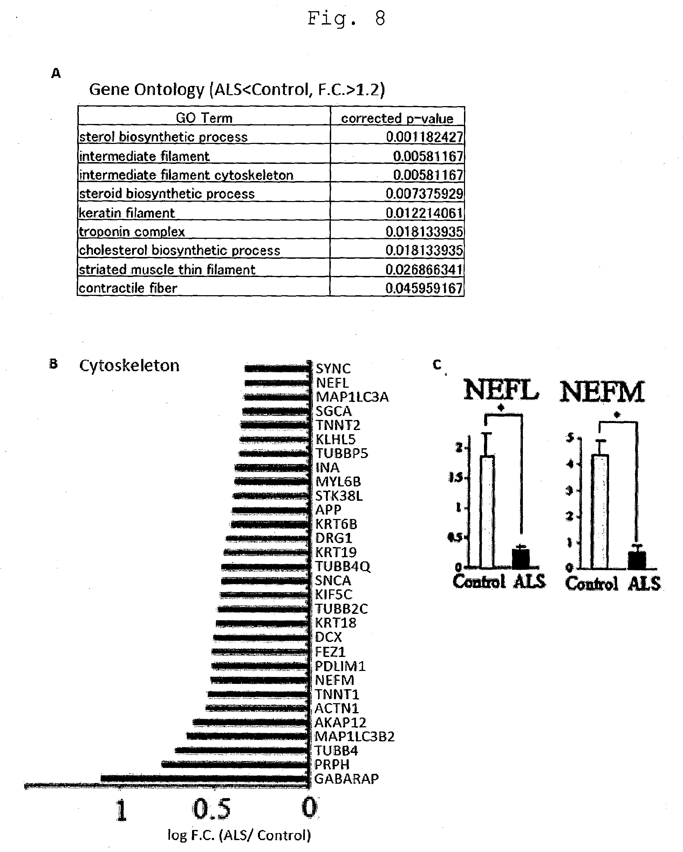

Fig. 8A shows the category of the gene showing less expression in ALS-iPS cell-derived neurons. Fig. 8B shows the ratio of the expression level of each cytoskeleton-related gene in ALS-iPS cell-derived neurons relative to that of the control. Fig. 8C shows the measurement results by quantitative PCR of NEFL and NEFM mRNAs in neurons derived from each iPS cell.

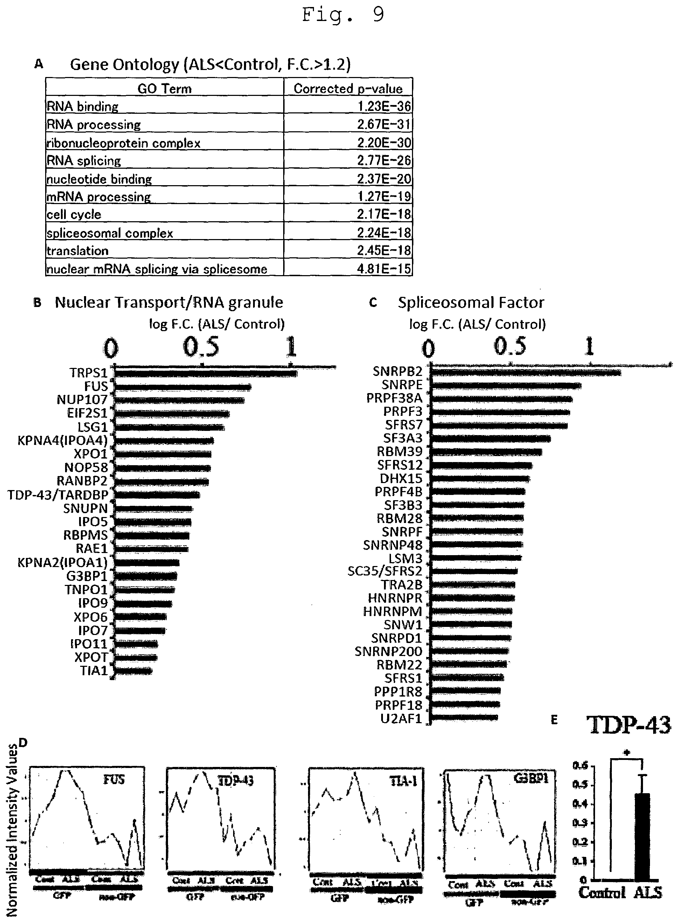

Fig. 9A shows the category of the gene showing high expression in ALS-iPS cell-derived neurons. Fig. 9B shows the ratio of the expression level of each gene related to nuclear transport/RNA granule in ALS-iPS cell-derived neurons relative to that of the control. Fig. 9C shows the ratio of the expression level of each spliceosome-related gene in ALS-iPS cell-derived neurons relative to that of the control. Fig. 9D shows the measurement results of FUS, TDP-43, TIA1 and G3BP1 mRNAs by quantitative PCR. Fig. 9E shows the measurement results of TDP-43 mRNA by quantitative PCR.

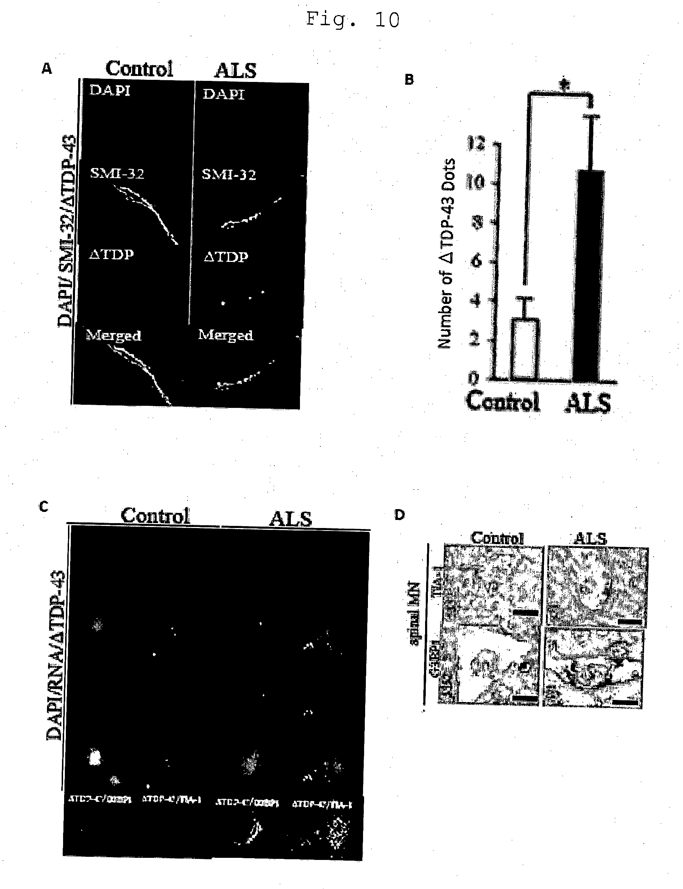

Fig. 10A shows co-stained (ΔTDP-43 and SMI-32, motor neuron markers) images of iPS cell-derived neurons introduced with HB9::tdTomato-ΔTDP-43. Fig. 10B shows the measurement results of the number of dots of ΔTDP-43 aggregates. Fig. 10C shows co-stained images of ΔTDP-43 and RNA (penultimate), the lower panel shows co-stained images with ΔTDP-43 and G3BP1 (left panel and 3rd from the left) and co-stained images with ΔTDP-43 and TIA1 (2nd from the left and right panel), of iPS cell-derived neurons introduced with HB9::tdTomato-ΔTDP-43. Fig. 10D shows stained images of TIA1 (upper panel) and G3BP1 (bottom panel) in sporadic ALS patients-derived spinal motor neurons.

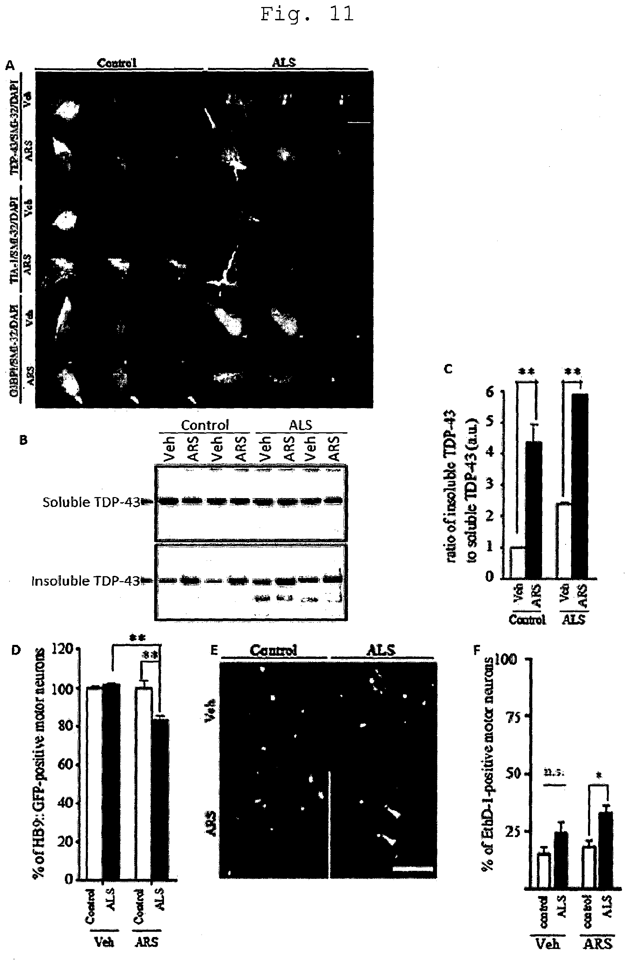

Fig. 11A shows immunostained images (red) of TDP-43, TIA-1 and G3BP1 of iPS cell-derived neurons of the arsenite (ARS) addition group and the control group. Figs. 11B and C show the results of two repeats of measurement of the amounts of TDP-43 protein contained in the soluble component and the insoluble component when the iPS cell-derived neurons of the arsenite (ARS) addition group and the control group were dissolved in 1% Triton X-100 solution. Figs. 11D and E show the measurement results of the GFP-positive cell number of the iPS cell-derived neurons introduced with HB9::GFP of the arsenite (ARS) addition group and the control group and fluorescence microscopic images thereof. Fig. 11F shows the measurement results of the EthD-1-positive cell number when EthD-1 was added to the iPS cell-derived neurons of the arsenite (ARS) addition group and the control group.

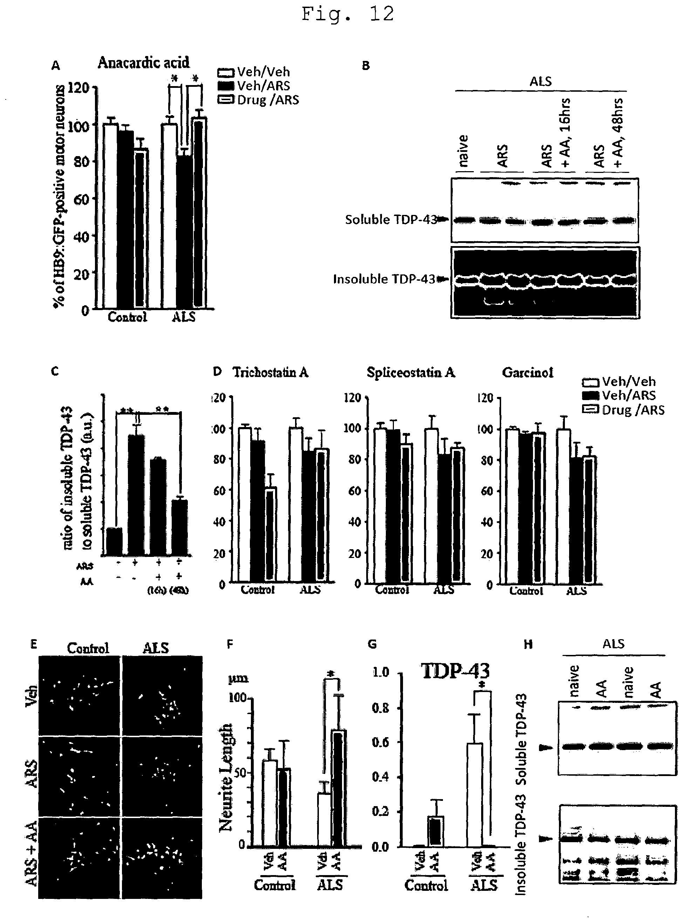

Fig. 12A shows the measurement results of the GFP-positive cell number of the iPS cell-derived neurons introduced with HB9::GFP of the control group, arsenite addition group and arsenite and anacardic acid addition group. Figs. 12B and C are graphs showing the measurement results of the amounts of TDP-43 protein contained in the soluble component and the insoluble component when the iPS cell-derived neurons of the control group, arsenite addition group, arsenite and anacardic acid 16 hr addition group, and arsenite and anacardic acid 48 hr addition group were dissolved in 1% Triton X-100 solution, and the ratio thereof. Fig. 12D shows the measurement results of the GFP-positive cell number of the iPS cell-derived neurons introduced with HB9::GFP of the control group, arsenite addition group and arsenite and drug addition group (Trichostatin A, Spliceostatin A and Garcinol). Fig. 12E shows the fluorescence microscopic images of the GFP-positive cell number of the iPS cell-derived neurons introduced with HB9::GFP of the control group, arsenite addition group and arsenite and anacardic acid addition group. Fig. 12F shows the measurement results of the length of neurite in GFP-positive cell of the iPS cell-derived neurons introduced with HB9::GFP of the control group and anacardic acid addition group. Fig. 12G shows the measurement results by quantitative PCR of the mRNA amount of TDP-43 in the GFP-positive cell of the iPS cell-derived neurons introduced with HB9::GFP of the control group and anacardic acid addition group. Fig. 12H shows the measurement results of the TDP-43 protein amount contained in the soluble component and the insoluble component when GFP-positive cell of iPS cell-derived neurons introduced with HB9::GFP was dissolved in 1% Triton X-100 solution in the control group and anacardic acid addition group.

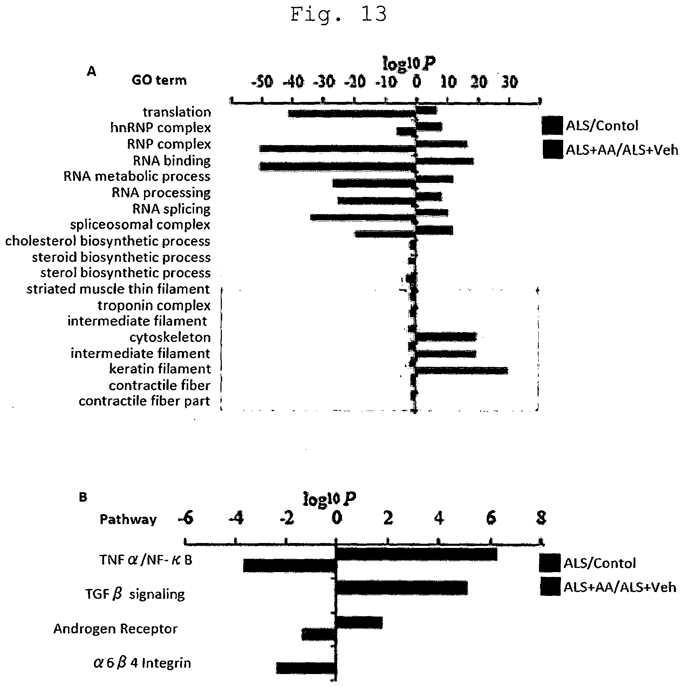

Fig. 13A shows the results of comparison of the category of genes expressed in control iPS cell-derived neurons and ALS-iPS cell-derived neurons (red) and the results of comparison of the category of genes expressed in ALS-iPS cell-derived neuron non-addition group and ALS-iPS cell-derived neuron anacardic acid addition group. Fig. 13B shows the results of comparison of respective signal transduction related genes expressed in control iPS cell-derived neurons and ALS-iPS cell-derived neurons (red) and the results of comparison of respective signal transduction related genes expressed in the ALS-iPS cell-derived neuron non-addition group and ALS-iPS cell-derived neuron anacardic acid addition group.

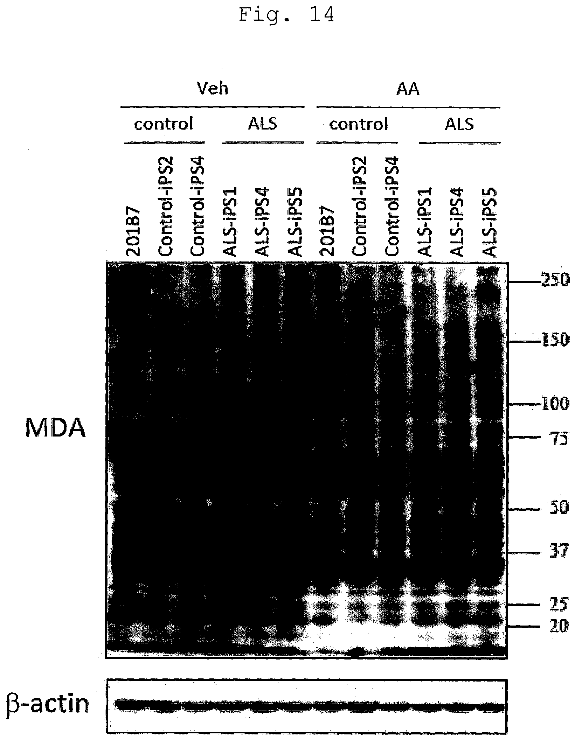

Fig. 14 shows the measurement results of the amount of MDA (malondialdehyde)-modified protein in the total protein extracted from neurons derived from each iPS cell in the control group (Veh) and the anacardic acid addition group (AA).

### DETAILED DESCRIPTION OF THE INVENTION

Disclosed herein is a method of screening for a prophylactic and/or therapeutic drug for ALS, using a neuron obtained by inducing differentiation of an ALS iPS cell, preferably, an ALS patients-derived iPS cell having a mutation in TDP-43, and a prophylactic and/or therapeutic agent for ALS, containing an anacardic acid derivative identified by the screening method (hereinafter sometimes to be referred to as a prophylactic and/or therapeutic agent for ALS).

In the present specification, "iPS cell of amyotrophic lateral sclerosis (ALS)" is used to mean not only an iPS cell established from a somatic cell derived from an animal (preferably human) affected with ALS, but also an iPS cell established from a somatic cell derived from an animal (preferably human) unaffected with ALS and having a gene polymorphism associated with the disease.

### I. Production of iPS cells

Induced pluripotent stem (iPS) cell is an artificial stem cell derived from a somatic cell, which can be produced by introducing a specific reprogramming factor in the form of a DNA or protein into a somatic cell, and show almost equivalent property (e.g., pluripotent differentiation and proliferation potency based on self-renewal) as ES cells (K. Takahashi and S. Yamanaka (2006) Cell, 126:663-676; K. Takahashi et al. (2007), Cell, 131:861-872; J. Yu et al. (2007), Science, 318:1917-1920; Nakagawa, M. et al., Nat. Biotechnol. 26:101-106 (2008); WO2007/069666). The reprogramming factor may be constituted with a gene specifically expressed by ES cell, a gene product or non-coding RNA thereof, a gene playing an important role for the maintenance of undifferentiation of ES cell, a gene product or non-coding RNA thereof, or a low molecular weight compound. Examples of the gene contained in the reprogramming factor include Oct3/4, Sox2, Sox1, Sox3, Sox15, Sox17, Klf4, Klf2, c-Myc, N-Myc, L-Myc, Nanog, Lin28, Fbx15, ERas, ECAT15-2, Tel1, beta-catenin, Lin28b, Sall1, Sall4, Esrrb, Nr5a2, Tbx3, Glis1 and the like. These reprogramming factors may be used alone or in combination. Examples of the combination of the reprogramming factors include combinations described in WO2007/069666, WO2008/118820, WO2009/007852, WO2009/032194, WO2009/058413, WO2009/057831, WO2009/075119, WO2009/079007, WO2009/091659, WO2009/101084, WO2009/101407, WO2009/102983, WO2009/114949, WO2009/117439, WO2009/126250, WO2009/126251, WO2009/126655, WO2009/157593, WO2010/009015, WO2010/033906, WO2010/033920, WO2010/042800, WO2010/050626, WO2010/056831, WO2010/068955, WO2010/098419, WO2010/102267, WO2010/111409, WO2010/111422, WO2010/115050, WO2010/124290, WO2010/147395, WO2010/147612, Huangfu D, et al. (2008), Nat. Biotechnol., 26: 795-797, Shi Y, et al. (2008), Cell Stem Cell, 2: 525-528, Eminli S, et al. (2008), Stem Cells. 26:2467-2474, Huangfu D, et al. (2008), Nat Biotechnol. 26:1269-1275, Shi Y, et al. (2008), Cell Stem Cell, 3, 568-574, Zhao Y, et al. (2008), Cell Stem Cell, 3:475-479, Marson A, (2008), Cell Stem Cell, 3, 132-135, Feng B, et al. (2009), Nat Cell Biol. 11:197-203, R.L. Judson et al., (2009), Nat. Biotech., 27:459-461, Lyssiotis CA, et al. (2009), Proc Natl Acad Sci USA. 106:8912-8917, Kim JB, et al. (2009), Nature. 461:649-643, Ichida JK, et al. (2009), Cell Stem Cell. 5:491-503, Heng JC, et al. (2010), Cell Stem Cell. 6:167-74, Han J, et al. (2010), Nature. 463:1096-100, Mali P, et al. (2010), Stem Cells. 28:713-720, and Maekawa M, et al. (2011), Nature. 474:225-9.

Examples of the above-mentioned reprogramming factor include, but are not limited to, factors used for enhancing the establishment efficiency, such as histone deacetylase (HDAC) inhibitors [e.g., low-molecular inhibitors such as valproic acid (VPA), trichostatin A, sodium butyrate, MC 1293, and M344, nucleic acid-based expression inhibitors such as siRNAs and shRNAs against HDAC (e.g., HDAC1 siRNA Smartpool® (Millipore), HuSH 29mer shRNA Constructs against HDAC1 (OriGene) and the like), and the like], MEK inhibitor (e.g., PD184352, PD98059, U0126, SL327 and PD0325901), Glycogen synthase kinase-3 inhibitor (e.g., Bio and CHIR99021), DNA methyl transferase inhibitors (e.g., 5-azacytidine), histone methyl transferase inhibitors [for example, low-molecular inhibitors such as BIX-01294, and nucleic acid-based expression inhibitors such as siRNAs and shRNAs against Suv39hl, Suv39h2, SetDB1 and G9a], L-channel calcium agonist (e.g., Bayk8644), butyric acid, TGFβ inhibitor or ALK5 inhibitor (e.g., LY364947, SB431542, 616453 and A-83-01), p53 inhibitor (e.g., siRNA and shRNA against p53), ARID3A inhibitor (e.g., siRNA and shRNA against ARID3A), miRNA such as miR-291-3p, miR-294, miR-295, mir-302 and the like, Wnt Signaling activating agent (e.g., soluble Wnt3a), neuropeptide Y, prostaglandins (e.g., prostaglandin E2 and prostaglandin J2), hTERT, SV40LT, UTF1, IRX6, GLIS1, PITX2, DMRTB1 and the like. In the present specification, these factors used for enhancing the establishment efficiency are not particularly distinguished from the reprogramming factor.

When in the form of a protein, a reprogramming factor may be introduced into a somatic cell by a method, for example, lipofection, fusion with cell penetrating peptide (e.g., TAT derived from HIV and polyarginine), microinjection and the like.

When in the form of a DNA, a reprogramming factor may be introduced into a somatic cell by the method of, for example, vector of virus, plasmid, artificial chromosome and the like, lipofection, liposome, microinjection and the like. Examples of the virus vector include retrovirus vector, lentivirus vector (Cell, 126, pp.663-676, 2006; Cell, 131, pp.861-872, 2007; Science, 318, pp.1917-1920, 2007), adenovirus vector (Science, 322, 945-949, 2008), adeno-associated virus vector, vector of Hemagglutinating Virus of Japan (WO 2010/008054) and the like. Examples of the artificial chromosome vector include human artificial chromosome (HAC), yeast artificial chromosome (YAC), bacterial artificial chromosome (BAC, PAC) and the like. As the plasmid, plasmids for mammalian cells can be used (Science, 322:949-953, 2008). The vector can contain regulatory sequences of promoter, enhancer, ribosome binding sequence, terminator, polyadenylation site and the like so that a nuclear reprogramming substance can be expressed and further, where necessary, a selection marker sequence of a drug resistance gene (e.g., kanamycin resistance gene, ampicillin resistance gene, puromycin resistance gene and the like), thymidine kinase gene, diphtheria toxin gene and the like, a reporter gene sequence of green fluorescent protein (GFP), β glucuronidase (GUS), FLAG and the like, and the like. Moreover, the above-mentioned vector may have a LoxP sequence before and after thereof to simultaneously cut out a gene encoding a reprogramming factor or a gene encoding a reprogramming factor bound to the promoter, after introduction into a somatic cell.

When in the form of RNA, for example, it may be introduced into a somatic cell by a means of lipofection, microinjection and the like, and RNA incorporating 5-methylcytidine and pseudouridine (TriLink Biotechnologies) may be used to suppress degradation (Warren L, (2010) Cell Stem Cell. 7:618-630).

Examples of the culture medium for inducing iPS cell include 10 to 15% FBS-containing DMEM, DMEM/F12 or DME culture medium (these culture media can further contain LIF, penicillin/streptomycin, puromycin, L-glutamine, nonessential amino acids, β-mercaptoethanol and the like as appropriate) or a commercially available culture medium [for example, culture medium for mouse ES cell culture (TX-WES culture medium, Thromb-X), culture medium for primate ES cell (culture medium for primate ES/iPS cell, Reprocell), serum-free medium (mTeSR, Stemcell Technologies)] and the like.

Examples of the culture method include contacting a somatic cell with a reprogramming factor on 10% FBS-containing DMEM or DMEM/F12 culture medium at 37°C in the presence of 5% CO₂ and culturing for about 4 to 7 days, thereafter reseeding the cells on feeder cells (e.g., mitomycin C-treated STO cells, SNL cells etc.), and culturing the cells in a bFGF-containing culture medium for primate ES cell from about 10 days after the contact of the somatic cell and the reprogramming factor, whereby ES-like colonies can be obtained after about 30 to about 45 days or longer from the contact.

Alternatively, the cells are cultured on feeder cells (e.g., mitomycin C-treated STO cells, SNL cells etc.) at 37°C in the presence of 5% CO₂ in a 10% FBS-containing DMEM culture medium (which can further contain LIF, penicillin/streptomycin, puromycin, L-glutamine, nonessential amino acids, β-mercaptoethanol and the like as appropriate), whereby ES-like colonies can be obtained after about 25 to about 30 days or longer. Desirably, a method using a somatic cell itself to be reprogrammed, or an extracellular substrate (e.g., Laminin-5 (WO2009/123349) and Matrigel (BD)), instead of the feeder cells (Takahashi K, et al. (2009), PLoS One. 4:e8067 or WO2010/137746) .

Besides the above, a culture method using a serum-free medium can also be recited as an example (Sun N, et al. (2009), Proc Natl Acad Sci USA. 106:15720-15725). Furthermore, to enhance establishment efficiency, an iPS cell may be established under hypoxic conditions (oxygen concentration of not less than 0.1% and not more than 15%) (Yoshida Y, et al. (2009), Cell Stem Cell. 5:237-241 or WO2010/013845), can be mentioned.

The culture medium is exchanged with a fresh culture medium once a day between the above-mentioned cultures, from day 2 from the start of the culture. While the cell number of the somatic cells used for nuclear reprogramming is not limited, it is about 5×10³ to about 5×10⁶ cells per 100 cm² culture dish.

The iPS cell can be selected based on the shape of the formed colony. When a drug resistance gene which is expressed in association with a gene (e.g., Oct3/4, Nanog) expressed when a somatic cell is reprogrammed is introduced as a marker gene, an established iPS cell can be selected by culturing in a culture medium (selection culture medium) containing a corresponding drug. When the marker gene is a fluorescent protein gene, iPS cell can be selected by observation with a fluorescence microscope, when it is a luminescent enzyme gene, iPS cell can be selected by adding a luminescent substrate, and when it is a chromogenic enzyme gene, iPS cell can be selected by adding a chromogenic substrate.

The term "somatic cells" used in the present specification means any animal cells (preferably, cells of mammals inclusive of human) excluding germ line cells and totipotent cells such as ovum, oocyte, ES cells and the like. Somatic cells unlimitatively encompass any of somatic cells of fetuses, somatic cells of neonates, and mature healthy or pathogenic somatic cells, and any of primary cultured cells, passage cells, and established lines of cells. Specific examples of the somatic cells include (1) tissue stem cells (somatic stem cells) such as neural stem cell, hematopoietic stem cell, mesenchymal stem cell, dental pulp stem cell and the like, (2) tissue progenitor cells, (3) differentiated cells such as lymphocyte, epithelial cell, endothelial cell, myocyte, fibroblast (skin cells etc.), hair cell, hepatocyte, gastric mucosal cell, enterocyte, splenocyte, pancreatic cell (pancreatic exocrine cell etc.), brain cell, lung cell, renal cell and adipocyte and the like, and the like.

In the present disclosure, the choice of mammalian individual from which somatic cells are collected is not particularly limited, but it is preferably a human. More preferably, it is desirable that the somatic cells be collected from a patient with ALS (particularly, sporadic ALS), or a non-ALS patient or healthy person having a gene polymorphism that associates with the disease. Here, the gene polymorphisms include, but are not limited to, polymorphisms with a mutation in the coding region of TDP-43. As the mutation of TDP-43, for example, mutation of conversion of the 343rd glutamine or 337th methionine or 298th glycine, preferably mutation of substitution of the 343rd glutamine with other amino acid, preferably arginine, and mutation of substitution of the 337th glutamine with other amino acid, preferably valine, and mutation of substitution of the 298th glutamine with other amino acid, preferably serine, in the amino acid sequence of TDP-43 can be mentioned.

### II. Method for inducing differentiation into neurons

In the present disclosure, neuron refers to a cell expressing at least HB9, preferably a motor neuron expressing HB9 and SMI-32.

The method of inducing the differentiation from the above-described iPS cells to neurons is not particularly limited; useful methods include differentiation by high-density culture on a fibroblast feeder layer (JP-A-2008-201792), differentiation by co-cultivation with stromal cells (SDIA method) (e.g., WO2001/088100, WO/2003/042384), differentiation by suspension culture (SFEB method) (WO2005/123902) and a combination thereof.

As other aspect, an adherent culture method wherein an iPS cell is adhered to a culture dish after a coating treatment and cultivated in any medium containing various appropriate additives can be mentioned.

Examples of coating agents to be used for the adherent culture method include Matrigel, collagen, gelatin, poly-L-lysine, poly-D-lysine, fibronectin, laminin and combinations thereof, with preference given to a combination of poly-L-lysine and laminin.

As a medium to be used for the adherent culture method, a basal medium containing appropriate additives can be mentioned, which is not particularly limited as long as it can be used for cultivation of animal cells. Such basal medium includes, for example, Neurobasal medium, Neural Progenitor Basal medium, NS-A medium, BME medium, BGJb medium, CMRL 1066 medium, Glasgow MEM medium, Improved MEM Zinc Option medium, IMDM, Medium 199, Eagle MEM, αMEM, DMEM, DMEM/F12 medium, Ham's medium, RPMI 1640 medium, Fischer's medium, and mixed medium thereof, with preference given to a mixture of Neurobasal medium and DMEM/F12. As the additive, serum, serum replacement (KSR) (Invitrogen), retinoic acid (RA), BMP inhibitor, TGF β family inhibitor, Sonic Hedgehog (SHH), bFGF (FGF-2), FGF-8, EGF, HGF, LIF, BDNF, GDNF, NT-3, amino acid, vitamin, interleukin, insulin, transferrin, heparin, heparan sulfate, collagen, fibronectin, progesterone, selenite, B27-supplement, N2-supplement, ITS-supplement, antibiotics and mercaptoethanol can be mentioned. Here, the BMP inhibitor is exemplified by Noggin, chordin, follistatin, Dorsomorphin LDN-193189 and the like. The TGF β family inhibitor is exemplified by SB431542, SB202190, SB505124, NPC30345, SD093, SD908, SD208, LY2109761, LY364947, LY580276, A-83-01 and the like. These additives can be used in an appropriate combination. As a preferable combination of additives, (1) Noggin and SB431542, (2) Noggin, B27-supplement and N2-supplement, (3) retinoic acid, Sonic Hedgehog, B27-supplement and N2-supplement and (4) BDNF, GDNF, NT-3, B27-supplement and N2-supplement can be mentioned. These combinations may be used in further combination.

The iPS cell concentration at the start of cultivation can be set as appropriate to allow neurons to be formed efficiently. The iPS cell concentration at the start of cultivation is not particularly limited, and is, for example, about 1×10 to about 1×10⁶ cells/ml, preferably about 1×10⁴ to about 5×10⁵ cells/ml.

Other culturing conditions such as culturing temperature and CO₂ concentration can be set as appropriate. The culturing temperature is not particularly limited, and is, for example, about 30 to 40°C, preferably about 37°C. The CO₂ concentration is, for example, about 1 to 10%, preferably about 5%.

As other aspect, a modified SFEBq method wherein a cell is adhered to a coating-treated culture dish after neurosphere formation and cultivated in any medium containing various appropriate additives can be mentioned.

For neurosphere formation in the modified SFEBq method, a non-cell-adhesive culture vessel is preferable. A useful non-cell-adhesive culture vessel is a culture vessel whose surface is not treated to artificially increase adhesion of cells thereto (e.g., coating with extracellular matrix and the like), or a culture vessel treated to artificially suppress adhesion of cells thereto [e.g., coating with polyhydroxyethyl methacrylate (poly-HEMA)], or a culture vessel after a coating treatment with Lipidure (NOF) can be used. Preferably, the coating agent is Lipidure.

To form neurosphere in the modified SFEBq method, a cell can be cultivated using the above-mentioned basal medium and additives. As a preferable medium, a mixture of DMEM/F12 or a Neurobasal medium can be mentioned. As a combination of preferable additives, (1) Dorsomorphin and SB431542 and (2) retinoic acid, Sonic Hedgehog, FGF-2 and B27-supplement can be mentioned. These combinations may be used in further combination.

The cell concentration at the time of the start of the neurosphere formation can be appropriately set to enable efficient formation of neurosphere. While the cell concentration at the time of the start of the culture is not particularly limited, for example, it is about 1×10⁴ to about 5×10⁶ cells/ml, preferably about 5×10⁵ to about 2×10⁶ cells/ml.

To adhere the neurosphere obtained by the modified SFEBq method to a culture dish, any of the aforementioned coating agents can be used, with preference given to Matrigel.

In addition, when an adherent culture is performed in the modified SFEBq method, the aforementioned basal medium and additive can be used therefor. As a preferable medium, Neurobasal medium can be mentioned. As a combination of preferable additives, BDNF, GDNF and NT-3 can be mentioned.

In the modified SFEBq method, other culturing conditions such as temperature and CO₂ concentration can be set as appropriate. The culturing temperature is not particularly limited, and is, for example, about 30 to 40°C, preferably about 37°C. The CO₂ concentration is, for example, about 1 to 10%, preferably about 5%.

The thus-induced neurons can be purified by an expression marker of a neural stem cell such as HB9 and the like.

### III. Screening method for prophylactic or therapeutic drug for amyotrophic lateral sclerosis

Disclosed herein is a method of screening for a candidate substance of a prophylactic and/or therapeutic drug for amyotrophic lateral sclerosis, which includes contacting an iPS cell-derived neuron of ALS obtained by the aforementioned method with a test compound, and using each index.

### <Insoluble TDP-43>

In one aspect, the method of screening for a candidate substance of a prophylactic and/or therapeutic drug for amyotrophic lateral sclerosis of the present disclosure (hereinafter to be referred to as a screening method of the present disclosure) includes the following steps:
(1) contacting a neuron induced to differentiate from an iPS cell of amyotrophic lateral sclerosis with a test compound,
(2) measuring the protein amount of TDP-43 in said neuron, which is insoluble in a detergent solution, and
(3) selecting, as a candidate prophylactic and/or therapeutic drug for amyotrophic lateral sclerosis, a test compound that decreases the protein amount of said insoluble TDP-43 by comparison with a control not contacted with the test compound.

In the present disclosure, a detergent to be contained in a detergent solution used to confirm the solubility of TDP-43 is a non-ionic detergent and, for example, N,N-Bis(3-D-gluconamidopropyl)cholamide (BIGCHAP), N,N-Bis(3-D-gluconamidopropyl)deoxycholamide (Deoxy-BIGCHAP), NIKKOL BL-9EX (Polyoxyethylene(9)Lauryl Ether), Octanoyl-N-methylglucamide (MEGA-8), noyl-N-methylglucamide (MEGA-9), Decanoyl-N-methylglucamide (MEGA-10), Polyoxyethylene(8)Octylphenyl Ether (Triton X-114), Polyoxyethylene(9)Octylphenyl Ether (NP-40), Polyoxyethylene(10)Octylphenyl Ether (Triton X-100), Polyoxyethylene(20)Sorbitan Monolaurate (Tween 20), Polyoxyethylene(20)Sorbitan Monopalmitate (Tween 40), Polyoxyethylene(20)Sorbitan Monostearate (Tween 60), Polyoxyethylene(20)Sorbitan Monooleate (Tween 80), Polyoxyethylene(20)Sorbitan Trioleate, Polyoxyethylene(23)Lauryl Ether (Brij35), Polyoxyethylene(20)Cethyl Ether (Brij58), n-Dodecyl-β-D-maltopyranoside, n-Heptyl-β-D-thioglucopyranoside, n-Octyl-β-D-glucopyranoside, n-Octyl-β-D-thioglucopyranoside, n-Nonyl-β-D-thiomaltoside, IGEPAL CA-630, Digitonin or Saponin, from Soybeans can be recited as examples. More preferable detergent is polyoxyethylene alkyl ether, which is exemplified by Polyoxyethylene(10)Octylphenyl Ether (Triton X-100).

In the present disclosure, while the amount of the detergent to be contained in the detergent solution is not particularly limited, it is 0.5% to 2%, preferably 1%.

Insoluble TDP-43 protein can be detected by dissolving a protein insoluble in the above-mentioned solution in a cell lysis solution containing SDS, and according to a method well known to those of ordinary skill in the art, for example, immunoassay methods such as ELISA (enzyme linked immunosorbent assay) and Western blotting (immunoblotting).

In the present disclosure, SNRPB2 or hnRNPA1 protein insoluble in the above-mentioned solution may be subjected to the measurement instead of the TDP-43 protein.

In the present disclosure, to easily detect insoluble TDP-43, neurons induced to differentiate from iPS cells of amyotrophic lateral sclerosis may be contacted with a test compound, and further contacted with oxidative agent, like as arsenite or hydrogen peroxide. The preferable oxidative agent is arsenite. In the case of arsenite, the concentration of arsenite to be added may by 0.1 mM to 1 mM, preferably 0.25 mM or 0.5 mM. While the time of addition is not particularly limited, contact for at least 1 hr is preferable.

### <Cytoskeleton-related gene>

According to one aspect, the screening method of the present disclosure includes the following steps of:
(1) contacting a neuron induced to differentiate from an iPS cell of amyotrophic lateral sclerosis with a test compound,
(2) measuring the expression level of a cytoskeleton-related gene in said neuron, and
(3) selecting, as a candidate prophylactic and/or therapeutic drug for amyotrophic lateral sclerosis, a test compound that increases the expression level of said cytoskeleton-related gene by comparison with a control not contacted with the test compound.

In the present disclosure, the cytoskeleton-related gene refers to a gene encoding a protein constituting cytoskeleton, namely, a protein constituting actin fiber, microtubule and intermediate filament. For example, the genes described in Table 1 can be recited as examples.

**Table 1: Cytoskeleton-related gene**

| gene name | Accession No. |
|---|---|
| SYNC | NM_001161708 |
| NEFL | NM_006158 |
| MAP1LC3A | NM 032514 |
| SGCA | NM_000023 |
| TNNT2 | NM_000364 |
| KLHL5 | NM_001007075 |
| TUBBP5 | NR_027156 |
| INA | NM_032727 |
| MYL6B | NM_001199629 |
| STK38L | NM_015000 |
| APP | NM_000484 |
| KRT6B | NM_005555 |
| DRG1 | NM_004147 |
| KRT19 | NM_002276 |
| TUBB4Q | U83110 |
| SNCA | NM_000345 |
| KIF5C | NM_004522 |
| TUBB2C | NM_006088 |
| KRT18 | NM_000224 |
| DCX | NM_000555 |
| FEZ1 | NM_005103 |
| PDLIM1 | NM_020992 |
| NEFM | NM_001105541 |
| TNNT1 | NM_003283 |
| ACTN1 | NM_001102 |
| AKAP12 | NM_005100 |
| MAP1LC3B2 | NM_001085481 |
| TUBB4 | NM_001197181 |
| PRPH | NM_006262 |

The expression level of the cytoskeleton-related gene can be measured by a method well known to those of ordinary skill in the art, which includes, for example, Northern blotting, RT-PCR method, *in situ* hybridization and the like.

### <RNA splicing or RNA synthesis related gene>

According to one aspect, the screening method of the present disclosure includes the following steps of:
(1) contacting a neuron induced to differentiate from an iPS cell of amyotrophic lateral sclerosis with a test compound,
(2) measuring the expression level of an RNA-binding gene or RNA splicing-related gene in said neuron, and
(3) selecting a test compound that decreases the expression level of said RNA-binding gene or RNA splicing-related gene by comparison with a control not contacted with the test compound.

In the present disclosure, the RNA-binding gene refers to a gene encoding a protein or nucleic acid which is selectively bound to an RNA molecule by a non-covalent binding, and the RNA splicing-related gene refers to a gene encoding an RNA and protein constituting a spliceosome necessary for removing intron from an mRNA precursor transcribed from DNA. For example, the genes described in Table 2 and Table 3 can be recited as examples.

**Table 2: RNA-binding gene**

| gene name | Accession No. |
|---|---|
| TRPS1 | NM_014112 |
| FUS | NM_004960 |
| NUP107 | NM_020401 |
| EIF2S1 | NM_004094 |
| LSG1 | NM_018385 |
| KPNA4 (IPOA4) | NM_002268 |
| XPO1 | NM_003400 |
| NOP58 | NM_015934 |
| RANBP2 | NM_006267 |
| TDP-43/TARDBP | NM_007375 |
| SNUPN | NM_001042581 |
| IPO5 | NM_002271 |
| RBPMS | NM_001008710 |
| RAE1 | NM_001015885 |
| KPNA2(IPOA1) | NM_002266 |
| G3BP1 | NM_005754 |
| TNPO1 | NM_002270 |
| IPO9 | NM_018085 |
| XPO6 | NM_015171 |
| IPO11 | NM_001134779 |
| IPO7 | NM_006391 |
| XPOT | NM_007235 |
| TIA1 | NM_022037 |

**Table 3: RNA splicing-related gene**

| gene name | Accession No. |
|---|---|
| SNRPB2 | NM_003092 |
| SNRPE | NM_003094 |
| PRPF38A | NM_032864 |
| PRPF3 | NM_004698 |
| SFRS7 | NM_001031684 |
| SF3A3 | NM_006802 |
| RBM39 | NM_001242599 |
| SFRS12 | NM_001077199 |
| DHX15 | NM_001358 |
| PRPF4B | NM_003913 |
| SF3B3 | NM_012426 |
| RBM28 | NM_001166135 |
| SNRPF | NM_003095 |
| SNRNP48 | NM_152551 |
| LSM3 | NM 014463 |
| SC35/SFRS2 | NM_001195427 |
| TRA2B | NM_004593 |
| HNRNPR | NM_001102397 |
| HNRNPM | NM_005968 |
| SNW1 | NM_012245 |
| SNRPD1 | NM_006938 |
| SNRNP200 | NM_014014 |
| RBM22 | NM_018047 |
| SFRS1 | NM_006924 |
| PPP1R8 | NM_002713 |
| PRPF18 | NM_003675 |
| U2AF1 | NM_006758 |

The expression levels of the RNA-binding gene and RNA splicing-related gene can be measured by a method well known to those of ordinary skill in the art, which includes, for example, Northern blotting, RT-PCR method, *in situ* hybridization and the like.

### <Aggregates of TDP-43 fragments>

According to one aspect, the screening method of the present disclosure includes the following steps of:
(1) contacting a neuron induced to differentiate from an iPS cell of amyotrophic lateral sclerosis, which is introduced with a TDP-43 fragment, with a test compound,
(2) measuring the number of aggregates of the TDP-43 fragments, and
(3) selecting, as a candidate prophylactic and/or therapeutic drug for amyotrophic lateral sclerosis, a test compound that decreases said number of aggregates by comparison with a control not contacted with the test compound.

In the present disclosure, the TDP-43 fragment may be any fragment of TDP-43 protein as long as it functions as a nucleus of aggregation in neurons and shows the properties of promoting formation of aggregates. Preferred is a protein containing an amino acid sequence obtained by referring to the NCBI accession number of TDP-43/TARDBP described in Table 2 less a nuclear transport signal (NLS) (78th - 84th amino acids) and 187th - 192nd amino acids, which contains the sequence described in SEQ ID NO: 2. It may be a fusion protein with a fluorescent protein which enables confirmation of the introduced TDP-43 fragment.

While the method of introducing a TDP-43 fragment into a cell is not particularly limited, for example, the following method can be used.

When a TDP-43 fragment is introduced in the form of a protein, for example, techniques such as lipofection, fusion with cell penetrating peptide (e.g., TAT derived from HIV and polyarginine), microinjection and the like can be used for introduction into the neuron.

On the other hand, when a TDP-43 fragment is introduced in the form of a DNA, for example, a vector of virus, plasmid, artificial chromosome and the like may be introduced into iPS cell or neuron by a method such as lipofection, liposome, microinjection and the like. Examples of the virus vector include retrovirus vector, lentivirus vector, adenovirus vector, adeno-associated virus vector, vector of Hemagglutinating Virus of Japan and the like. Examples of the artificial chromosome vector include human artificial chromosome (HAC), yeast artificial chromosome (YAC), bacterial artificial chromosome (BAC, PAC) and the like. Examples of the plasmid include plasmids for mammalian cells can be used. The vector can contain regulatory sequences of promoter, enhancer, ribosome binding sequence, terminator, polyadenylation site and the like so that a mRNA containing the nucleotide sequence shown in SEQ ID NO: 1 can be expressed and further, where necessary, selection marker sequences of a drug resistance gene (e.g., kanamycin resistance gene, ampicillin resistance gene, puromycin resistance gene and the like), thymidine kinase gene, diphtheria toxin gene and the like, a reporter gene sequence of fluorescent protein, β glucuronidase (GUS), FLAG and the like, and the like. As a promoter, SV40 promoter, LTR promoter, CMV (cytomegalovirus) promoter, RSV (Rous sarcoma virus) promoter, MoMuLV (Moloney mouse leukemia virus) LTR, HSV-TK (herpes simplex virus thymidine kinase) promoter, EF-α promoter, CAG promoter and TRE promoter (minimal CMV promoter having a Tet response element with continuous 7 tetO sequences). When a TRE promoter is used, a fusion protein of tetR and VP16AD or a fusion protein of reverse tetR (rtetR) and VP16AD is desirably expressed simultaneously in the same cell. Here, a vector having a TRE promoter and capable of expressing a fusion protein of reverse tetR (rtetR) and VP16AD is referred to as a drug responsive inducible vector. In addition, to introduce an expression cassette consisting of a promoter and SEQ ID No: 1 bonded thereto into a chromosome of a target cell and cut it out as necessary therefrom, the above-mentioned vector may have a transposon sequence before and after the expression cassette. While the transposon sequence is not particularly limited, piggyBac can be mentioned.

When a TDP-43 fragment is introduced in the form of RNA, for example, techniques such as electroporation, lipofection, microinjection and the like can be used for introduction into the neuron.

The aggregates obtained by intracellular aggregation of the thus-introduced TDP-43 fragments can be counted by visual observation of aggregates showing fluorescence using a fluorescence microscope, by staining them with an anti-TDP-43 antibody or using a TDP-43 fragment fused with a fluorescent protein. Alternatively, the aggregates can be counted by IN Cell Analyzer 2000 (GE Healthcare).

In this case, the aggregates co-stained with TIA-1 or G3BP1 (stress granule marker) may be counted.

### <Number of survival neurons>

According to one aspect, the screening method of the present disclosure includes the following steps of:
(1) contacting a neuron induced to differentiate from an iPS cell of amyotrophic lateral sclerosis with a test compound,
(2) further contacting the neuron with arsenite,
(3) measuring the number of survival neurons in (2), and
(4) selecting, as a candidate prophylactic and/or therapeutic drug for amyotrophic lateral sclerosis, a test compound that increases said survival number by comparison with a control not contacted with the test compound.

In the present disclosure, the concentration of arsenite to be added may by 0.1 mM to 1 mM, preferably 0.25 mM or 0.5 mM. While the time of addition is not particularly limited, contact for at least 1 hr is preferable.

While the method of measuring the number of survival neurons is not particularly limited and can be performed by a method widely used by those of ordinary skill in the art, a method including measurement of absorbance by MTT method, WST-1 method, WST-8 method, and a method including staining with TO (thiazole orange), PI (propidium iodide), 7AAD, calcein AM and/or ethidium homodimer 1 (EthD-1), and counting by a flow cytometer can be mentioned.

### <Oxidative stress>

According to one aspect, the screening method of the present disclosure includes the following steps of:
(1) contacting a neuron induced to differentiate from an iPS cell of amyotrophic lateral sclerosis with a test compound,
(2) measuring the oxidative stress of the neuron, and
(3) selecting a test compound that suppresses said oxidative stress by comparison with a control not contacted with the test compound.

In the present disclosure, the oxidative stress means an imbalance between an intracellular oxidation reaction and an antioxidant reaction and inclination toward the former state. In the present screening method, the oxidative stress of the neurons can be evaluated by, for example, the level of oxidation of the intracellular protein, lipid or DNA, and preferably, can be evaluated by the amount of malondialdehyde which is a lipoperoxide that modifies intracellular proteins. Here, the amount of malondialdehyde (MDA) can be detected as an MDA-modified protein resulting from the reaction with an amino group in the lysine residue or terminal amino group in the protein. For example, as modification to an amino group, N-propenal and dihydropyridine adducts having a carbonyl structure can be mentioned.

Such MDA-modified protein is recognized by an anti-MDA antibody, and can be measured by a method well known to those of ordinary skill in the art, for example, immunoassay methods such as ELISA (enzyme linked immunosorbent assay) and Western blotting (immunoblotting).

### <Test compound>

Test compounds can also be obtained using any one of many approaches in combinatorial library methods known in the art including (1) a biological library method, (2) a synthetic library method using deconvolution, (3) a "one-bead one-compound" library method, and (4) a synthetic library method using affinity chromatography selection. An example of the biological library method using affinity chromatography selection is limited to a peptide library method, however, the other 4 approaches can be applied to peptides, non-peptide oligomers, or low-molecular-weight compound libraries (Lam (1997) Anticancer Drug Des. 12: 145-67). Examples of a method for synthesizing a molecular library can be found in the art (DeWitt et al. (1993) Proc. Natl. Acad. Sci. U.S.A. 90: 6909-13; Erb et al. (1994) Proc. Natl. Acad. Sci. U.S.A. 91: 11422-6; Zuckermann et al. (1994) J. Med. Chem. 37: 2678-85; Cho et al. (1993) Science 261: 1303-5; Carell et al. (1994) Angew. Chem. Int. Ed. Engl. 33: 2059; Carell et al. (1994) Angew. Chem. Int. Ed. Engl. 33: 2061; Gallop et al. (1994) J. Med. Chem. 37: 1233-51). Compound libraries can be constructed in the form of solutions (see Houghten (1992) Bio/Techniques 13: 412-21) or beads (Lam (1991) Nature 354: 82-4), chips (Fodor (1993) Nature 364: 555-6), bacteria (U.S. Patent No. 5,223,409), spores (U.S. Patent No. 5,571,698, U.S. Patent No. 5,403,484, and U.S. Patent No. 5,223,409), plasmids (Cull et al. (1992) Proc. Natl. Acad. Sci. U.S.A. 89: 1865-9), or phages (Scott and Smith (1990) Science 249: 386-90; Devlin (1990) Science 249: 404-6; Cwirla et al. (1990) Proc. Natl. Acad. Sci. U.S.A. 87: 6378-82; Felici (1991) J. Mol. Biol. 222: 301-10; U.S. Patent Application No. 2002103360).

### IV. Prophylactic and/or therapeutic agent for amyotrophic lateral sclerosis

The present invention provides an anacardic acid derivative for use in the prophylaxis and/or treatment of amyotrophic lateral sclerosis.

In the present invention, it is desirable that the amyotrophic lateral sclerosis be sporadic amyotrophic lateral sclerosis or familial amyotrophic lateral sclerosis having a mutation of TDP-43.

In the present invention, the anacardic acid derivative means a compound represented by the formula 1: 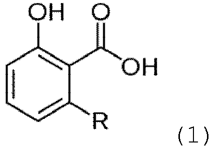 wherein R is C10-17 saturated or unsaturated hydrocarbon group.

The C10-17 saturated or unsaturated hydrocarbon group for R may be a straight chain structure or a branched structure, and may contain a conjugated double bond. Said anacardic acid derivative optionally has one or more substituents at substitutable position(s) on the formula 1. Examples of the substituent include halogen (chlorine atom, fluorine atom, iodine atom, bromine atom), amino group, hydroxyl group, carbonyl group and the like.

The anacardic acid derivative to be used in the present invention is more preferably an anacardic acid of the formula 1 wherein R is a pentadecyl group or a pentadecyl-8,11,14-trienyl group.

An anacardic acid derivative can also be extracted from cashew oil by the method described in JP-A-H8-217720, or purchased from Leancare Ltd. or INDOFINE Chemical Company, Inc. Where necessary, a desired anacardic acid derivative can be obtained by appropriately modifying a commercially available anacardic acid derivative by a method generally employed in the field.

The prophylactic and/or therapeutic agent for ALS of the present invention can be administered orally or parenterally in the form of the active ingredient anacardic acid derivative as it is alone, or as a pharmaceutical composition in an appropriate dosage form blended with a pharmacologically acceptable carrier, excipient, diluent and the like.

As the composition for oral administration, solid or liquid dosage forms, specifically tablets (including sugar-coated tablets and film-coated tablets), pills, granules, powders, capsules (including soft capsules), syrups, emulsions, suspensions and the like can be mentioned. Meanwhile, as examples of the composition for parenteral administration, injections, suppositories and the like are used; the injections may include dosage forms such as intravenous injections, subcutaneous injections, intracutaneous injections, intramuscular injections and drip infusion injections. These preparations are produced by a well-known method using additives, including excipients (e.g., organic excipients like sugar derivatives such as lactose, sucrose, glucose, mannitol, and sorbitol; starch derivatives such as cornstarch, potato starch, α starch, and dextrin; cellulose derivatives such as crystalline cellulose; gum arabic; dextran; and pullulan; and inorganic excipients like silicate derivatives such as light anhydrous silicic acid, synthetic aluminum silicate, calcium silicate, and magnesium aluminometasilicate; phosphates such as calcium hydrogen phosphate; carbonates such as calcium carbonate; and sulfates such as calcium sulfate), lubricants (e.g., stearic acid, stearic acid metal salts such as calcium stearate and magnesium stearate; talc; colloidal silica; waxes such as beeswax and spermaceti; boric acid; adipic acid; sulfates such as sodium sulfate; glycol; fumaric acid; sodium benzoate; DL leucine; lauryl sulfates such as sodium lauryl sulfate and magnesium lauryl sulfate; silicates such as silicic anhydride and silicic acid hydrates; and the aforementioned starch derivatives), binders (e.g., hydroxypropylcellulose, hydroxypropylmethylcellulose, polyvinylpyrrolidone, macrogol, and the same compounds as the aforementioned excipients), disintegrants (e.g., cellulose derivatives such as low-substituted hydroxypropylcellulose, carboxymethylcellulose, carboxymethylcellulose calcium, and internally crosslinked carboxymethylcellulose sodium; chemically modified starches and celluloses such as carboxymethylstarch, carboxymethylstarch sodium, and crosslinked polyvinylpyrrolidone), emulsifiers (e.g., colloidal clays such as bentonite and Veegum; metal hydroxides such as magnesium hydroxide and aluminum hydroxide; anionic detergents such as sodium lauryl sulfate and calcium stearate; cationic detergents such as benzalkonium chloride; and non-ionic detergents such as polyoxyethylene alkyl ethers, polyoxyethylene sorbitan fatty acid ester, and sucrose fatty acid ester), stabilizers (para-oxybenzoic acid esters such as methyl paraben and propyl paraben; alcohols such as chlorobutanol, benzyl alcohol, and phenylethyl alcohol; benzalkonium chloride; phenols such as phenol and cresol; thimerosal; dehydroacetic acid; and sorbic acid), taste/odor correctives (e.g., sweeteners, souring agents, and flavors in common use), and diluents.

The dose of the anacardic acid derivative as an active ingredient of the prophylactic and/or therapeutic agent for ALS in the present invention is variable according to the patient's symptoms, age, weight and other factors.

The dose differs depending on the symptoms, age and the like; at least 0.1 mg (suitably 0.5 mg) to at most 1000 mg (suitably 500 mg) per dose for oral administration, or at least 0.01 mg (suitably 0.05 mg) to at most 100 mg (suitably 50 mg) per dose for parenteral administration, can be administered to an adult 1 to 6 times a day. The dose may be increased or reduced according to the symptoms.

Furthermore, the prophylactic and/or therapeutic agent for ALS of the present invention may be used in combination with other drugs, for example, glutamic acid action suppressants (e.g., riluzole and the like), neurotrophic factors [e.g., insulin-like growth factor-1, 5-HT_{1A} receptor agonists (e.g., xaliproden) and the like] and the like. The prophylactic and/or therapeutic agent for ALS of the present invention and these other drugs can be administered simultaneously, sequentially, or separately.

The present invention is hereinafter described in further detail by means of the following Example.

### Examples

### Reference Example 1: Generation of iPS cell

Fibroblasts were established from the skin biopsied from two ALS patients with informed consent (ALS-fibro 1 and ALS-fibro 2) or the skin of 5 donors unaffected with ALS as a control (HDF1388, TIG107, Control-fibro 1, Control-fibro 2 and Control-fibro 3). Into these fibroblasts were respectively introduced 4 factors (Oct3/4, Sox2, Klf4 and c-Myc) or 3 factors (Oct3/4, Sox2 and Klf4) by the method described in Takahashi, K. et al., Cell, 131(5), 861, 2007 to establish iPS cells (201B7, Control-iPS1, Control-iPS3, Control-iPS6, ALS-iPS1, ALS-iPS2 and ALS-iPS3). Furthermore, Oct3/4, Sox2, Klf4, L-Myc, Lin28 and p53 shRNA were introduced by the method described in Okita K, et al., Nat Methods. 8(5), 409, 2011 to establish iPS cells (Control-iPS2, Control-iPS4, Control-iPS5, ALS-iPS4, ALS-iPS5 and ALS-iPS6). These cell lines are shown in Table 4. The phase contrast microscopic images of Control-iPS4 and ALS-iPS1 and the stained images of non-differentiation markers (Nanog and SSEA-4) are shown in Fig. 1A. In addition, the mutation of Q343R, M337V and G298S of TDP-43 was confirmed in the fibroblasts derived from two ALS patients (ALS-fibro 1, ALS-fibro 2 and ALS-fibro 3), respectively (Fig. 1B).

**Table 4: Cell line**

| Fibroblast | iPS cell line | Reprogramming method |
|---|---|---|
| HDF1388 | 201B7 | Retrovirus vector (4 factors) |
| TIG107 | Control-iPS1 | Retrovirus vector (4 factors) |
| TIG107 | Control-iPS2 | Episomal vector (6 factors) |
| Control-fibro 1 | Control-iPS3 | Retrovirus vector (3 factors) |
| Control-fibro 2 | Control-iPS4 | Episomal vector (6 factors) |
| Control-fibro 2 | Control-iPS5 | Episomal vector (6 factors) |
| Control-fibro 3 | Control-iPS6 | Retrovirus vector (4 factors) |
| ALS-fibro 1 | ALS-iPS1 | Retrovirus vector (4 factors) |
| ALS-fibro 1 | ALS-iPS2 | Retrovirus vector (4 factors) |
| ALS-fibro 1 | ALS-iPS3 | Retrovirus vector (4 factors) |
| ALS-fibro 2 | ALS-iPS4 | Episomal vector (6 factors) |
| ALS-fibro 2 | ALS-iPS5 | Episomal vector (6 factors) |
| ALS-fibro 2 | ALS-iPS6 | Episomal vector (6 factors) |
| ALS-fibro 3 | ALS-iPS7 | Episomal vector (6 factors) |
| ALS-fibro 3 | ALS-iPS8 | Episomal vector (6 factors) |
| ALS-fibro 3 | ALS-iPS9 | Episomal vector (6 factors) |

Of these established iPS cells, the expression of exogenous genes of 201B7, Control-iPS3, Control-iPS6, ALS-iPS1, ALS-iPS2 and ALS-iPS3 generated using a retrovirus was examined by quantitative PCR. As a result, the expression of the exogenous genes was confirmed to have decreased in all cell lines (Fig. 1C). In addition, in the iPS cells similarly generated using an episomal vector, it was confirmed that the vector was not incorporated into the chromosome.

Sequentially, the karyotype of these iPS cells was examined to find no chromosome abnormality. Methylation of CpG sequence present in the promoter regions of Nanog and Oct3/4 was examined by a bisulfite method to find not much difference in the level of methylation in respective iPS cells.

Furthermore, as a differentiation induction ability, teratoma was formed by subcutaneous transplantation to an immunodeficient mouse. As a result, a characteristic shape could be confirmed in all triploblastic tissues in the teratoma (Fig. 2).

### Reference Example 2: Generation of spinal motor neurons using spinal motor neuron induction (adherent culture method)

Induction of spinal motor neurons (MNs) by an adherent culture method was performed by slightly altering the method described in Wada et al., PLoS One. 4(8):e6722, 2009. In brief, iPS cells were separated into small masses, seeded in a dish coated with poly-L-lysine (Sigma-Aldrich) and Laminin (BD), and cultured for 10 days in a medium (N2B27 medium) containing 200 mM glutamine (Life Technologies), 0.5% N2 (Life Technologies) and 1% B27 (Life Technologies), added with Neurobasal medium A (Life Technologies) and DMEM/F12 (Life Technologies) at 1:1 and supplemented with 100 ng/ml recombinant Noggin (R&D Systems) and 10 mM SB431542 (Cayman Chemical). Furthermore, small masses were separated with 200 U/ml collagenase containing 1 mM CaCl₂, placed in a poly-L-lysine/Laminin-coated dish, and cultured for 7 days in a N2B27 medium containing 100 ng/ml Noggin. Then, they were separated with Accutase (Innovative Cell Technologies), placed in a poly-L-lysine/Laminin-coated dish, and cultured for 7 days in a N2B27 medium containing 1 mM retinoic acid (Sigma-Aldrich) and 100 ng/ml Sonic Hedgehog (R&D Systems), and continuously cultured in a N2B27 medium containing 10 ng/ml BDNF (R&D Systems), 10 ng/ml GDNF (R&D Systems) and 10 ng/ml NT-3 (R&D Systems) (Fig. 3A).

The obtained cells were seeded at 10,000 cells/cm² in a dish coated with poly-L-lysine, Laminin and Fibronectin (Millipore), cultured for 7 days in a N2B27 medium containing 10 ng/ml BDNF, 10 ng/ml GDNF and 10 ng/ml NT-3 and used. Changes in the MNs specific gene expression was confirmed on day 0, day 17, day 24 and day 41 from the start of differentiation induction. As a result, MNs were confirmed on day 41 (Fig. 3B). In addition, Islet-1, Tuj1 and HB9 positive cells were confirmed on day 35 (Fig. 3C).

### Reference Example 3: Analyses of insoluble TDP-43 and binding protein

MNs obtained by the above-mentioned adherent culture method were added with cytosine arabinoside (Sigma-Aldrich) and cultured for 3 days. The cells were recovered, dissolved in TS buffer (50 mM Tris-HCl buffer, pH 7.5, 0.15 M NaCl, 5 mM ethylenediaminetetraacetic acid, 5 mM ethylene glycol bis (β-aminoethyl ether)-N,N,N,N-tetraacetic acid, and protease inhibitor cocktail (Roche)) containing 1% Triton X-100, and separated into a soluble fraction and an insoluble fraction. A cell lysate containing 10 mg of the total protein was prepared for each fraction, and the TDP-43 amount was compared using SDS-PAGE. As a result, in MNs (ALS-MNs) induced from ALS patient-derived iPS cells, the protein amount of TDP-43 was shown to be significantly high in a fraction insoluble in a detergent (Fig. 4A). In this Example, the size of the TDP-43 fragments insoluble in Triton X-100 solution was 43, 35 or 25 kDa. From the above, it has been found that mutated TDP-43 relating to ALS is formed in a form in which the protein is insoluble in Triton X-100 solution in iPS cell-derived MNs.

In addition, it was confirmed that the amounts of SNRPB2 which is a component of U2 RNP and hnRNPA1 which is a nucleocytoplasmic shuttle protein increased in the insoluble fraction of the ALS-derived MNs, and these proteins were bound to TDP-43 (Figs. 4B and C).

Sequentially, a lentivirus vector having a sequence wherein GFP (HB9::GFP) was linked to the HB9 promoter described in Lee SK, et al., Development, 131, 3295, 2004 was introduced into the MNs, obtained by the above-mentioned adherent culture method, to generate MNs that express GFP in conjunction with the expression of HB9. Using the MNs, intracellular localization of SNRPB2, hnRNPA1 and TDP-43 was examined. As a result, it was confirmed that these proteins were co-localized in ALS-iPS cell-derived MNs (Fig. 5). This was also confirmed in MNs biopsied from ALS patients.

From these results, it is assumed that the properties of TDP-43 complex change due to the mutation of TDP-43 in MNs of ALS patients, which influences RNA splicing and nucleocytoplasmic shuttling.

### Reference Example 4: Generation of spinal motor neuron by using spinal motor neuron induction (modified SFEBq method)

Induction of MNs by a modified SFEBq method was performed by modifying the method described in Watanabe K, et al., Nat Neurosci, 8, 288, 2005. In brief, iPS cell was placed on a Lipidure-coated 96 well dish (NOF), and cultured for 12 days in a medium containing 5% KSR (Invitrogen), DMEM/Hams' F12 (Sigma-Aldrich), MEM-NEAA (Invitrogen), L-glutamine (Sigma-Aldrich) and 2-Mercaptoethanol, and supplemented with 2 µM Dorsomorphin and SB431542. Thereafter, the medium was changed to a Neurobasal medium (Gibco) added with B27, 1 µM retinoic acid, 100 to 500 ng/ml Sonic Hedgehog and 12.5 ng/ml FGF2 and the cells were cultured for 10 days. Sequentially, the obtained cells were seeded in a Matrigel (BD Biosciences)-coated dish, and adherent cultured in a Neurobasal medium added with 10 ng/ml BDNF, 10 ng/ml GDNF and 10 ng/ml NT-3 for 13 days. Furthermore, the cells were separated with Accutase, transferred to a Matrigel-coated 24 well plate at 500,000 cells/well and cultured for 15 days to induce MNs (Fig. 6A). It was confirmed by immunostaining that MNs derived from each iPS cell after induction showed positive to each neuronal marker (Tuj1 and Nestin) (Fig. 6B). When confirmed by quantitative PCR, the expression level of Tuj1 was inconsistent (Fig. 6C). Thus, using MNs introduced with the aforementioned lentivirus vector having HB9::GFP and showing fluorescence in conjunction with HB9 expression, HB9::GFP-positive cells purified by sorting by FACS (Fig. 7A) were examined by High-Content analysis using IN Cell Analyzer 2000 (GE Healthcare). As a result, short neurite was confirmed in ALS-MNs (Figs. 7B and C).

### Reference Example 5: Analysis of gene expression of ALS-MNs

A gene that specifically expresses or does not express in HB9::GFP-positive ALS-MNs induced by a modified SFEBq method was identified by a gene ontology method. As a result, it was confirmed that the expression of the intermediate filament cytoskeleton-related factors described in Figs. 8A and B decreased in ALS-MNs. Of such genes, NEFM and NEFL were measured for expression by quantitative PCR. As a result, it was confirmed that the expression significantly decreased in ALS-MNs (Fig. 8C). On the other hand, the transcription amount of RNA splicing and RNA synthesis related protein was confirmed to increase (Figs. 9A, B and C). Similarly, it was confirmed by quantitative PCR that the expression of FUS, TDP-43, TIA-1 and G3BP1 increased in ALS-MNs (Fig. 9D). From these findings, it is assumed that abnormality in RNA metabolism occurred in ALS-MNs. Furthermore, since the expression levels of TDP-43 and SNRPB2 were confirmed to have increased in ALS-MNs (Fig. 9E), it is assumed that autogenous control of TDP-43 and SNRPB2 collapsed in ALS-MNs and the expression thereof could not be controlled. However, it is assumed that the abnormal RNA metabolism by such RNA-binding proteins including TDP-43 does not produce fatal denaturation for MN, but a vicious cycle of property change of TDP-43 and abnormal RNA metabolism is produced by aging, which leads to denaturation of ALS-MNs.

### Reference Example 6: Observation of TDP-43 aggregation

The potential abnormality in ALS-MNs is considered to occur as an increase of the amount of unvisualizable insoluble TDP-43 in the cytoplasm, which does not exist as an aggregate. Such insoluble TDP-43 is assumed to aggregate in the cytosol of ALS-MNs, triggered by the aggregation nucleus, and be visualized. Therefore, a lentivirus vector having a sequence, wherein the TDP-43 C-terminal fragment (ΔTDP-43) described in Nonaka T, et al., FEBS Lett. 583: 394-400 (2009) which lacks the nuclear transport signal and the 187th-192nd amino acid residues, and a fluorescence marker tdTomato were linked to the above-mentioned HB9 promoter (HB9::tdTomato-ΔTDP-43), was introduced to allow overexpression of ΔTDP-43 as an aggregation nucleus in each MNs. The inclusion formed by ΔTDP-43 showed localization in the cytoplasm (Fig. 10A). The number of dots of ΔTDP-43 aggregates significantly increased in ALS-MNs as compared to the control MNs (Fig. 10B). Furthermore, RNA was stained using SYTO RNASelect Green Fluorescent Cell Stain (Invitrogen), and the protein was stained with anti-TIA-1 antibody (Protein Tech) or anti-G3BP1 antibody (Protein Tech). The tdTomato and these stained images were observed with a fluorescence microscope. As a result, the aggregation of ΔTDP-43 matched with RNA localization, and also partly co-localized with stress granule markers, TIA-1 and G3BP1, in ALS-MNs (Fig. 10C). This matches with the localization of TIA-1 and G3BP1 near the nucleus of MNs in sporadic ALS patients (Fig. 10D).

Sequentially, to examine the effect of RNA in the cytoplasm on TDP-43 aggregation and cell death of ALS-MNs, the influence of the addition of arsenite (ARS), which is a translation inhibitor and RNA granule formation inducing agent, on MNs was investigated. 0.5 mM arsenite was added to GFP-positive cells of ALS-MNs introduced with the aforementioned HB9::GFP, and promotion of RNA granule formation (Fig. 11A) and increase of insoluble TDP-43 (Figs. 11B and C) were confirmed. In addition, the number of the cells of ALS-MNs 1 hr after arsenite addition was counted using IN Cell Analyzer 2000. As a result, the number of survival ALS-MNs significantly decreased as compared to the non-addition group and normal MNs group (Figs. 11D and E). Furthermore, 0.2 M EthD-1 (Invitrogen) was added to each MNs 1 hr after arsenite addition, the cells were incubated for 30 min at room temperature, and the number of cells subjected to EthD-1-positive injury was counted. As a result, the number was significantly high in ALS-MNs of the arsenite addition group (Fig. 11F). These data suggest that RNA granules induced by arsenite isolated TDP-43 in the cytoplasm, and acquired the property to easily form toxic aggregation of mutated TDP-43 in ALS-MNs.

### Example 1: Study of effect of candidate agent for ALS treatment

To identify a candidate drug for the treatment of ALS, drugs known to act on transcription, histone acetylation and splicing were added 16 or 48 hr before arsenite addition (5 µM anacardic acid, 3 µM trichostatin A and 100 ng/ml splicesostatin A) or 24 hr before (5 µM garcinol), the cells were incubated and the behavior thereof was observed. As a result, anacardic acid (AA), which is a histone acetylation (HAT) inhibitor and has an antioxidant action, significantly suppressed cell death of ALS-MNs, which is associated with the increase of arsenite inductive insoluble TDP-43 (Figs. 12A, B and C). On the other hand, trichostatin A (HDAC inhibitor), splicesostatin A (splicing inhibitor) and garcinol (HAT inhibitor) did not show such action (Fig. 12D). It was confirmed that AA also significantly increased the length of neurite in ALS-MNs (Figs. 12E and F), decreased the expression level of TDP-43 mRNA, and decreased the amount of insoluble TDP-43 (Figs. 12G and H). Furthermore, it was confirmed that AA addition decreased the factors relating to translation, RNP complex, RNA binding, RNA metabolism pathway, RNA processing, RNA splicing and spliceosomal complex, and increased the factors relating to intermediate filament cytoskeleton, intermediate filament and keratin filament (Fig. 13A). Furthermore, signal transduction pathway was analyzed. As a result, it was confirmed that AA addition decreased TNFα/NF-κB, andorogen receptor and α6β4 integrin pathways (Fig. 13B).

Sequentially, to confirm an antioxidant action of AA, the amount of the protein modified with MDA (malondialdehyde) in the total protein was confirmed by Western blotting using 5 to 15% gradient gel in the AA addition group and the AA non-addition group of control MNs or ALS-MNs. As a result, it was confirmed that ALS-MNs contained a large amount of MDA-modified protein, and the AA addition decreased the amount (Fig. 14). This tendency was particularly strong in proteins having a size of about 30 kDa to 50 kDa. This suggests that an oxidative stress is constantly present in ALS-MNs, and the oxidative stress can be suppressed by AA.

From the above results, it is assumed that ALS is developed by a continuous series of vicious cycle of (1) denaturation of RNA-binding protein, (2) abnormal RNA metabolism and (3) increase in oxidative stress. Therefore, each gene and phenomenon relating thereto becomes an index to substitute the ALS pathology.

## Claims

1. An anacardic acid derivative for use in the prophylaxis and/or treatment of amyotrophic lateral sclerosis,
wherein the anacardic acid derivative is a compound represented by the formula 1: 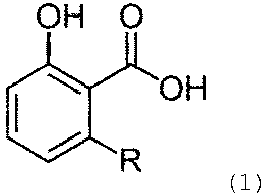 wherein R is C10-17 saturated or unsaturated hydrocarbon group.

2. The anacardic acid derivative for use according to claim 1, wherein the amyotrophic lateral sclerosis is sporadic amyotrophic lateral sclerosis.

3. The anacardic acid derivative for use according to claim 1, wherein the amyotrophic lateral sclerosis is accompanied by a mutation of TDP-43.

## Patentansprüche

1. Anacardsäurederivat zur Verwendung bei der Prophylaxe und/oder Behandlung von amyotropher Lateralsklerose,
wobei das Anacardsäurederivat eine Verbindung ist, die durch die Formel 1 dargestellt wird: 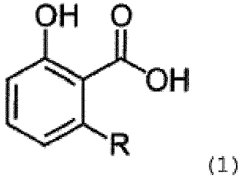 wobei R eine gesättigte oder ungesättigte C₁₀₋₁₇-Kohlenwasserstoffgruppe ist.

2. Anacardsäurederivat zur Verwendung gemäß Anspruch 1, wobei die amyotrophe Lateralsklerose eine sporadische amyotrophe Lateralsklerose ist.

3. Anacardsäurederivat zur Verwendung gemäß Anspruch 1, wobei die amyotrophe Lateralsklerose von einer Mutation von TDP-43 begleitet ist.

## Revendications

1. Dérivé d'acide anacardique pour une utilisation dans la prophylaxie et/ou le traitement de la sclérose latérale amyotrophique,
lequel dérivé d'acide anacardique est un composé représenté par la formule 1 : 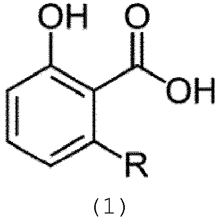 dans laquelle R est un groupe hydrocarboné saturé ou insaturé en C₁₀ à C₁₇.

2. Dérivé d'acide anacardique pour une utilisation selon la revendication 1, dans lequel ladite sclérose latérale amyotrophique est une sclérose latérale amyotrophique sporadique.

3. Dérivé d'acide anacardique pour une utilisation selon la revendication 1, dans lequel ladite sclérose latérale amyotrophique s'accompagne d'une mutation de TDP-43.
